# EUROPEAN PATENT APPLICATION

(11) **EP 3 915 543 A1**
(43) Date of publication of application: **01.12.2021**
(21) Application number: 20176977.5
(22) Date of filing: 28.05.2020
(51) Int. Cl.: A61K 9/107, A61K 9/14, A61K 31/519, A61K 47/32

(54) **SOLID SELF-NANOEMULSIFYING DRUG DELIVERY SYSTEM (S-SNEDDS)**

(71) Applicant: Evonik Operations GmbH, 45128 Essen (DE)
(72) Inventor: SCHMIED, Fabian-Pascal, 64285 Darmstadt (DE); BERNHARDT, Alexander, 65462 Ginsheim-Gustavsburg (DE); ENGEL, Andrea, 35205 Birmingham, AL, (US); MOERS, Christian, 63225 Langen (DE); ENDRES, Thomas, 35226 Hoover, AL, (US)
(74) Representative: Evonik Patent Association

(57) **Abstract**

The present invention refers to a pharmaceutical composition, comprising
(i) at least one methacrylic copolymer comprising units derived from methacrylamide;
(ii) at least one pharmaceutically active ingredient;
(iii) at least one lipid component; and
(iv) at least one surfactant. Furthermore, the invention refers to the pharmaceutical composition of the present invention for use as a medicament. Finally, the present invention refers to a specific method of preparing a solid self-nanoemulsifying drug delivery system and the solid self-nanoemulsifying drug delivery system obtained thereof.

## Description

### Field of the invention

The present invention refers to a pharmaceutical composition, comprising
(i) at least one methacrylic copolymer comprising units derived from methacrylamide;
(ii) at least one pharmaceutically active ingredient;
(iii) at least one lipid component; and
(iv) at least one surfactant. Furthermore, the invention refers to the pharmaceutical composition of the present invention for use as a medicament. Finally, the present invention refers to a specific method of preparing a solid self-nanoemulsifying drug delivery system and the solid self-nanoemulsifying drug delivery system obtained thereof.

### Background

Self-nanoemulsifying drug delivery systems (SNEDDS) are well known in the field of pharmaceutical compositions. The systems use the emulsion of poorly water-soluble active ingredients to improve drug solubilization. In general, two kinds of SNEDDS are known, liquid or semi-liquid SNEDDS and solid SNEDDS (S-SNEDDS). While the liquid or semi-liquid character of SNEDDS is often seen as a disadvantage in view of dosing for peroral applications and in view of their lack of storage stability, solid self-nanoemulsifying systems are preferred.

General methods of providing solid self-nanoemulsifying drug delivery systems or in the neighboring field of self-microemulsifying drug delivery systems (SMEDDS) are for example disclosed in:
EP 2 101 729 B1, which describes in this regard several ways for the conversion of microemulsions into the solid state. These are adsorption on colloidal silicon dioxide, stabilization of individual phases with colloidal silicon dioxide and hydrophobic colloidal silicon dioxide, incorporation in polyethylene glycol dispersions and spray drying.

Silva, D. Luis Antonio, et al. (International Journal of Pharmaceutics 541 (2018) 1 - 10), which describes a preparation of a solid self-microemulsifying drug delivery system (S-SMEDDS) by hot melt extrusion. S-SMEDDS were prepared by blending carvedilol and a lipid mixture with hydroxyl propyl methyl cellulose acetate succinate (HPMCAS). Extrudates prepared at the lowest drug concentration and highest temperature and recirculation time promoted a complete and rapid drug release at pH 6.8.

CN107308133 A, which describes S-SMEDDS comprising curcumin containing SMEDDS employing AEROSIL® 200 as adsorbent.

The object of the present inventors was to provide a pharmaceutical composition based on methacrylic copolymers, which is suitable as basis for a S-SNEDDS and shows equal or improved drug release and stability characteristics compared to other types of polymers used as base polymer.

In this regard the inventors of the present invention surprisingly found that pharmaceutical compositions comprising at least one methacrylic copolymer comprising units derived from methacrylamide, in particular dimethylaminopropyl methacrylamide, as base polymer can solve the object.

In contrast, (meth)acrylic polymers like EUDRAGIT® E 100, which do not comprise units derived from methacrylamide, for example disclosed in CN 107308133 A1, are not suitable, since they lead to processing problems when trying to form the S-SNEDDS.

### Summary of the invention

Therefore, in a first aspect the present invention refers to a pharmaceutical composition, comprising
(i) at least one methacrylic copolymer comprising units derived from methacrylamide, preferably dimethylaminopropyl methacrylamide;
(ii) at least one pharmaceutically active ingredient;
(iii) at least one lipid component; and
(iv) at least one surfactant.

In a second aspect the present invention pertains to the pharmaceutical composition of the present invention for use as a medicament.

In a third aspect the present invention refers to a specific method of preparing a solid self-nanoemulsifying drug delivery system by hot melt extruding, spray drying, adsorbing, electrospinning, electro spraying, prilling by vibration, granulating or supercritical fluidising the components of the pharmaceutical composition of the present invention.

Finally, in a fourth aspect the present invention refers to a solid self-nanoemulsifying drug delivery system obtained by the method of the present invention.

### Description of the figures

Fig. 1: Dissolution profiles of Solid-SNEDDS incorporating celecoxib and celecoxib drug substance in 500 ml 0.1N HCI in USP apparatus II. Each value designates the mean ± S.D. (n = 3).
Fig. 2: Dissolution profiles of polymer and drug substance (PODS) incorporating celecoxib and celecoxib drug substance in 500 ml 0.1N HCI in USP apparatus II. Each value designates the mean ± S.D. (n = 3).
Fig. 3: Dissolution profiles of Solid-SNEDDS incorporating efavirenz and efavirenz drug substance in 500 ml 0.1N HCI in USP apparatus II. Each value designates the mean ± S.D. (n = 3).
Fig. 4: Dissolution profiles of PODS incorporating efavirenz and efavirenz drug substance in 500 ml 0.1N HCI in USP apparatus II. Each value designates the mean ± S.D. (n = 3).
Fig. 5: Dissolution profiles of Solid-SNEDDS incorporating fenofibrate and fenofibrate drug substance in 500 ml 0.1N HCI in USP apparatus II. Each value designates the mean ± S.D. (n = 3).
Fig. 6: Dissolution profiles of PODS incorporating fenofibrate and fenofibrate drug substance in 500 ml 0.1N HCI in USP apparatus II. Each value designates the mean ± S.D. (n = 3).
Fig. 7: Dissolution profiles of Solid-SNEDDS (after 3 months of storage) incorporating celecoxib and celecoxib drug substance in 500 ml 0.1N HCI in USP apparatus II. Each value designates the mean ± S.D. (n = 3).
Fig. 8: Dissolution profiles of PODS (after 3 months of storage) incorporating celecoxib and celecoxib drug substance in 500 ml 0.1N HCI in USP apparatus II. Each value designates the mean ± S.D. (n = 3).
Fig. 9: Correlation between drug release and M_{w} of "Dimethylaminopropyl methacrylamide-butyl methacrylate-methyl methacrylate copolymer (2:1:1 (ratios by weight)) (E-173 kDa, E-254 kDa, E-281 kDa, E-305 kDa)" regarding the drug substance celecoxib. The equation and correlation coefficient for the Solid-SNEDDS data is: y = -0.0011x² + 0.2194x + 96.553 (r² = 0.9969) and for the PODS data is: y = 0.0019x² - 1.442x + 268.77 (r² = 0.9984).
Fig. 10: Correlation between drug release and *T*_{g} of "Dimethylaminopropyl methacrylamide-butyl methacrylate-methyl methacrylate copolymer (2:1:1 (ratios by weight)) (E-173 kDa, E-254 kDa, E-281 kDa, E-305 kDa)" regarding the drug substance celecoxib. The equation and correlation coefficient for the Solid-SNEDDS data is: y = -0.0544x² + 6.1381x - 46.627 (r² = 0.9994) and for the PODS data is: y = 0.3088x² - 57.577x + 2690.2 (r² = 0.9999).
Fig. 11: Correlation between drug release and M_{w} of "Dimethylaminopropyl methacrylamide-butyl methacrylate-methyl methacrylate copolymer (2:1:1 (ratios by weight)) (E-173 kDa, E-254 kDa, E-281 kDa, E-305 kDa)" regarding the drug substance efavirenz. The equation and correlation coefficient for the Solid-SNEDDS data is: y = -0.0052x² + 1.9413x - 75.651 (r² = 0.9315) and for the PODS data is: y = -0.0013x² - 0.1487x + 89.971 (r² = 0.9787).
Fig. 12: Correlation between drug release and *T*_{g} of "Dimethylaminopropyl methacrylamide-butyl methacrylate-methyl methacrylate copolymer (2:1:1 (ratios by weight)) (E-173 kDa, E-254 kDa, E-281 kDa, E-305 kDa)" regarding the drug substance efavirenz. The equation and correlation coefficient for the Solid-SNEDDS data is: y = -0.4676x² + 73.923x - 2817.1 (r² = 0.9756) and for the PODS data is: y = 0.0133x² - 2.3858x + 342.57 (r² = 0.9516).
Fig. 13: Correlation between drug release and M_{w} of "Dimethylaminopropyl methacrylamide-butyl methacrylate-methyl methacrylate copolymer (2:1:1 (ratios by weight)) (E-173 kDa, E-254 kDa, E-281 kDa, E-305 kDa)" regarding the drug substance fenofibrate. The equation and correlation coefficient for the Solid-SNEDDS data is: y = -0.0032x² + 1.0836x - 2.4189 (r² = 0.9988) and for the PODS data is: y = 0.0016x² - 0.9196x + 134.97 (r² = 1).
Fig. 14: Correlation between drug release and *T*_{g} of "Dimethylaminopropyl methacrylamide-butyl methacrylate-methyl methacrylate copolymer (2:1:1 (ratios by weight)) (E-173 kDa, E-254 kDa, E-281 kDa, E-305 kDa)" regarding the drug substance fenofibrate. The equation and correlation coefficient for the Solid-SNEDDS data is: y = -0.2446x² + 37.029x - 1312.2 (r² = 0.9963) and for the PODS data is: y = 0.2022x² - 35.955x + 1598.2 (r² = 0.9990).

### Detailed description

The present invention refers to a pharmaceutical composition, comprising or consisting of
(i) at least one methacrylic copolymer comprising or consisting of units derived from methacrylamide, preferably alkyl methacrylamide or aminoalkyl methacrylamide, more preferably C₁₋₅ alkyl methacrylamide or dimethylamino C₁₋₅ alkyl methacrylamide or diethylamino C₁₋₅ alkyl methacrylamide,
   most preferably dimethylaminopropyl methacrylamide; and preferably, at least one alkyl methacrylate, more preferably at least one C₁₋₅ alkyl methacrylate, most preferably methyl methacrylate and butyl methacrylate;
(ii) at least one pharmaceutically active ingredient;
(iii) at least one lipid component;
(iv) at least one surfactant;
(v) optionally at least one solvent;
(vi) optionally at least one (meth)acrylic copolymer, different from (i); and
(vii) optionally at least one additive.

Furthermore, the present invention refers to a pharmaceutical composition according to the present invention for use as a medicament, wherein preferably the pharmaceutical composition enhances the solubility of the included pharmaceutically active ingredient compared to the pharmaceutically active ingredient alone in the treatment of a disease of a human or an animal subject.

Moreover, the present invention refers to a method of preparing a solid self-nanoemulsifying drug delivery system comprising or consisting of the steps:
providing a self-nanoemulsifying drug delivery system by mixing compounds (ii) to (iv) and optionally (v) according to the present invention, and then
applying the obtained self-nanoemulsifying drug delivery system on compound (i) and optionally compounds (vi) and (vii) according to the present invention by hot melt extrusion, spray drying, adsorption, electrospinning, electro spraying, prilling by vibration, granulation or supercritical fluidization, preferably by hot melt extrusion, more preferably by hot melt extrusion at a temperature of 140 to 180 °C, preferably of 145 to 170 °C, to obtain the solid self-nanoemulsifying drug delivery system.

Finally, the present invention refers to a solid self-nanoemulsifying drug delivery system obtained by the method of the present invention.

These and other aspects, embodiments, features, and advantages of the invention will become apparent to a person skilled in the art through the study of the following detailed description and claims. Any feature from one aspect of the invention can be used in any other aspect of the invention. Furthermore, it will readily be understood that the examples contained herein are intended to describe and illustrate the invention but not to limit the invention and that, in particular, the invention is not limited to these examples.

According to the present invention it is understood by the feature "units derived from" in view of the polymer that the polymer comprises units, which are obtained by polymerizing specific monomers, which are then contained in the polymer as those units. For example, units derived from methacrylamide means that monomers having a methacrylamide group suitable for polymerization are used. The feature includes methacrylamide as such as well as other monomers containing a methacrylamide group like dimethylaminopropyl methacrylamide.

Numerical ranges that are indicated in the format "from x to y" also include the stated values. If several preferred numerical ranges are indicated in this format, it is self-evident that all ranges that result from the combination of the various endpoints are also included.

"One or more", as used herein, relates to at least one and comprises 1, 2, 3, 4, 5, 6, 7, 8, 9 or more of the referenced species. Similarly, "at least one" means one or more, i.e. 1, 2, 3, 4, 5, 6, 7, 8, 9 or more. "At least one", as used herein in relation to any component, refers to the number of chemically different molecules, i.e. to the number of different types of the referenced species, but not to the total number of molecules. For example, "at least one surfactant" means that at least one type of molecule falling within the definition for a surfactant is used but that also two or more different types of surfactants falling within this definition can be present, but does not mean that only one or more molecules of one type of surfactant are present.

All percentages given herein in relation to the compositions or formulations relate to wt.-% relative to the total weight of the respective composition, if not explicitly stated otherwise.

"Essentially free of" according to the present invention with regard to compounds means that the compound can only be present in an amount, which does not influence the characteristics of the composition, in particular the respective compound is present in less than 3 wt.-%, preferably 1 wt.-%, more preferably 0.01 wt.-%, based on the total weight of the composition or is not present at all.

The weight average molecular weight M_{w} and the number average molecular weight Mₙ can be determined by GPC or SEC (Gel Permeation Chromatography or Size Exclusion Chromatography) analysis (see, for example, H.F. Mark et al., Encyclopaedia of Polymer Science and Engineering, 2nd Edition, Vol. 10, pages 1 ff., J. Wiley, 1989), preferably employing polystyrene standards.

The glass transition temperature *T*_{g} may be determined by DSC (Differential Scanning Calorimetry) analysis according to DIN EN ISO 11357-2:2013 (measurement without addition of plasticizer at a residual monomer content (ReMo) of less than 100 ppm, heating rate 10°C/min, nitrogen atmosphere).

The Z-Average particle size D_{z} may be determined by dynamic light scattering (DLS) according to ISO 22412:2017 "Particle size analysis - Dynamic light scattering (DLS)". The polydispersity index (PDI) is determined from a two-parameter fit to the correlation data (the cumulants analysis). The calculations used for the determination of PDI are defined in the ISO standard documents 22412:2017.

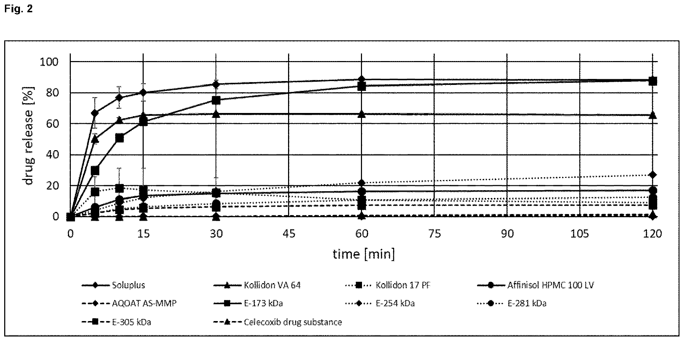

The amounts of polymerized monomer units in the copolymer and thus the monomer conversion rates are preferably determined by 1H-NMR spectroscopy. The method is well known to a skilled person in the field, for example as disclosed in US 8,399,523 B2, Fig. 2, Table 1, for 1H NMR spectroscopy of EUDRAGIT® E PO using deuterated MeOH_{*d*4}.

The amounts of polymerized monomer units may also be indirectly determined by the less elaborate determination of the residual monomer contents (ReMo) in the final copolymer preparation before removement of volatile substances, e.g. before drying (HPLC analysis regarding dimethylaminopropyl methacrylamide as well as GC analysis referring to butyl methacrylate and methyl methacrylate) and subsequent calculation. The method is well known to a skilled person in the field (see for instance WO 2012/048740 A1, p. 20).

### Methacrylic copolymer comprising units derived from methacrylamide (i)

According to the present invention any methacrylic copolymer, which comprises units derived from methacrylamide, preferably dimethylaminopropyl methacrylamide, which can be used in the field of pharmaceutical compositions is in general suitable. A person skilled in the field of methacrylic copolymers knows how to obtain such polymers, in particular by radical polymerization, preferably free radical polymerization.

In one embodiment the at least one methacrylic copolymer comprising units derived from methacrylamide has a weight average molecular weight M_{w} of from 10,000 to 500,000 g/mol, preferably 50,000 to 310,000 g/mol, more preferably 100,000 to 295,000 g/mol, more preferably 150,000 to 260,000 g/mol.

In one embodiment the at least one methacrylic copolymer comprising units derived from methacrylamide has a number average molecular weight Mₙ of from 15,000 to 100,000, preferably 40,000 to 85,000 g/mol.

In one embodiment the at least one methacrylic copolymer comprising units derived from methacrylamide has a polydispersity of from 2.0 to 5, preferably 3.5 to 4.5.

In one embodiment the at least one methacrylic copolymer comprising units derived from methacrylamide has a glass transition temperature *T*_{g} of from 65 to 100 °C, preferably 70 to 82 °C.

In one embodiment the at least one methacrylic copolymer is essentially free of reactive groups, like epoxy groups. In one embodiment the at least one methacrylic copolymer is not able to undergo further polymerization reactions.

In one embodiment the at least one methacrylic copolymer comprising units derived from methacrylamide is a dimethylaminopropyl methacrylamide-butyl methacrylate-methyl methacrylate copolymer, preferably comprising at least 34 wt.-%, more preferably 35 to 55 wt.-%, most preferably 45 to 50 wt.-%, of units derived from dimethylaminopropyl methacrylamide, based on the total weight of the copolymer.

In one embodiment, the at least one methacrylic copolymer comprising units derived from methacrylamide is obtained by radical polymerization, preferably free radical polymerization, of
40 to 60 wt.-%, preferably 45 to 55 wt.-%, of dimethylaminopropyl methacrylamide,
15 to 35 wt.-%, preferably 20 to 30 wt.-%, of butyl methacrylate; and
15 to 35 wt.-%, preferably 20 to 30 wt.-%, of methyl methacrylate, wherein the sum of the monomers is 100 wt.-%, in the presence of at least one initiator, preferably selected from di-(3,5,5)trimethylhexanoyl peroxide, tert-butyl peroxyneodecanoate, tert-butyl perbenzoate, tert-amyl peroxy-2-ethylhexanoate, bisdecanoyl peroxide, tert-butyl peroxy-2-ethylhexanoate, tert-butyl peroxy-2-ethylhexylcabonate, 1,1'-azobis(cyclohexanecarbonitrile), benzoyl peroxide, 2,2-di-(tert-butylperoxy)butane, dicumyl peroxide, di-tert-amyl peroxide, di-tert-butyl peroxide, lauroyl peroxide, tert-butylperoxy-3,5,5-trimethylhexanoate, 1,1-di-tert-butylperoxy-3,3,5-trimethylcyclohexane, 2-(1-cyan-1-methyl(ethyl)azocarboxamide, tert-butyl peroxyacetate, tert-butyl peroxypivalate or mixtures thereof, more preferably selected from tert-butyl peroxyneodecanoate and tert-butyl peroxypivalate or mixtures thereof;
and at least one chain-transfer agent preferably selected from carbon tetrachloride, carbon tetrabromide, bromotrichloromethane, 4-methylbenzenethiol, isooctyl 3-mercaptopropionate, pentaphenylethane, tert-nonyl mercaptan, 4,4'-thiobisbenzenethiol and n-dodecyl mercaptan, more preferably the chain-transfer agent is n-dodecyl mercaptan, and optionally in the presence of at least one solvent.

For the polymerization reaction any solvent which is suitable for use in those reactions is generally suitable.

When the initial amount of the dimethylaminopropyl methacrylamide in the monomer feed is known, the amounts (weight) of polymerized units of dimethylaminopropyl methacrylamide in the copolymer may be determined by the analysis of the total and individual residual monomer contents (ReMo) and can then be calculated accordingly. The total and individual residual monomer contents can be determined by High Pressure Liquid Chromatography (HPLC). The determination of the total and individual residual monomer contents by HPLC is well known to a skilled person.

The monomers dimethylaminopropyl methacrylamide, butyl methacrylate and methyl methacrylate are usually radically polymerized at different conversion rates in the reaction. The differences are depending on the polymerization conditions and radical polymerization method. The monomer conversion rate of butyl methacrylate and methyl methacrylate are comparably high and in the same range of about 95 wt.-% or more, more than 95 up to 99.9 wt.-% of polymerized monomers compared to the initial monomer feed. The monomer conversion rate of dimethylaminopropyl methacrylamide is comparably somewhat lower than that of butyl methacrylate and methyl methacrylate and about 85 wt.-% or more, 85 up to 95 wt.-% of polymerized monomers compared to the initial monomer feed. This means that when the weight ratio of dimethylaminopropyl methacrylamide:butyl methacrylate:methyl methacrylate is for instance 50:25:25 weight ratio in the initial monomer feed, the proportion of polymerized units in the copolymer may differ for instance by 47:26:27. The conversion rates also depend on the polymerization conditions such as temperature and duration of the reaction as well as the choice of polymerization initiator and chain-transfer-agent.

A preferred copolymer is dimethylaminopropyl methacrylamide-butyl methacrylate-methyl methacrylate copolymer polymerized from 40 to 60 wt.-%, preferably 45 to 55 wt.-% of dimethylaminopropyl methacrylamide, 15 to 35 wt.-%, preferably 20 to 30 wt.-% of butyl methacrylate and 15 to 35 wt.-%, preferably 20 to 30 wt.-% of methyl methacrylate, whereby the sum of the monomers is 100 wt.-%; wherein the amount of polymerized units of dimethylaminopropyl methacrylamide in the copolymer is 34 wt.-% or more, 34 to 60 or 40 to 57 wt.-% based on the total weight of the copolymer. Preferably, the amounts of polymerized units in the copolymer are 45 to 55 wt.-% of dimethylaminopropyl methacrylamide, 23 to 28 wt.-% of butyl methacrylate, and 23 to 28 wt.-% of methyl methacrylate.

The dimethylaminopropyl methacrylamide-butyl methacrylate-methyl methacrylate copolymer can preferably be prepared by radical polymerization, in particular free radical polymerization, in the presence of at least one polymerization initiator and at least one chain-transfer-agent. The monomer conversion rate of the monomers is preferably 85 wt.-% or more for dimethylaminopropyl methacrylamide, 95 wt.-% or more for butyl methacrylate and 95 wt.-% or more for methyl methacrylate. These monomer conversion rates can for example be achieved, when tert-butyl peroxyneodecanoate and tert-butyl peroxypivalate are used as polymerization initiators and n-dodecylmercaptan is used as chain-transfer-agent, for example in the presence of the solvent n-propanol and for example at a polymerization temperature of from 50 to 80 °C.

An exemplary process for the preparation of dimethylaminopropyl methacrylamide-butyl methacrylate-methyl methacrylate copolymer is as follows. The monomers dimethylaminopropyl methacrylamide (e.g. 500.0 g), butyl methacrylate (e.g. 250.0 g) and methyl methacrylate (e.g. 250.0 g) are added in a mixing equipment, for instance into a 3,000 ml round-bottom flask equipped with a magnetic bar, a return condenser and a nitrogen inlet. The reaction vessel is placed in a preheated water bath at 70 to 90 °C, e.g. 82 °C. When reaching an internal temperature of about 60 to 70 °C, e.g. 65 °C, n-dodecyl mercaptan (e.g. 3.0 g, 5.0 g, 9.0 g or 15.0 g) is added to control the molecular weight of the polymer during the process, while a solution of 0.1 to 1 wt.-% (calculated on weight of the monomers) of tert-butyl peroxyneodecanoate (e.g. 6.0 g) and 80 to 120 wt.-% (calculated on weight of the monomers) n-propanol (e.g.1,000 g) is added, for instance with a continuous flow rate of 2 to 10 g/min (e.g., 5 g/min) to initiate a radical polymerization. After about 2 to 4 hours, e.g. 3 h, at a temperature between 80 - 85 °C, 0.1 to 1 wt.-% (calculated on weight of the monomers) of a second initiator, e.g. tert-butyl peroxypivalate (e.g. 0.5 g) is used to complete the reaction, for instance for 60 to 120 min, e.g. for 90 min. The mixture may be cooled down and may be transferred to an oven at 30 to 50 °C, e.g. 40 °C, in order to remove n-propanol, e.g. over 48 h. The polymer may be purified using deionized water and may be dried afterwards, e.g. over 10 days at 50 °C. The dry, coarse polymer may be ground and milled (e.g. mesh size: 0.25 mm) using an Ultra Centrifugal Mill ZM 200 from Retsch GmbH (Haan, Germany).

The at least one methacrylic copolymer can be used in powder form, preferably having an average particle size D_{z} in the range of from 1 to 1,000 µm, more preferably from 100 to 500 µm. The powder can be obtained by milling and grinding.

### Pharmaceutically active ingredient (ii)

Any pharmaceutically active ingredient or mixtures of pharmaceutically active ingredients known to the skilled person may be incorporated in the pharmaceutical compositions and S-SNEDDS. However, the pharmaceutical compositions of the present invention are very useful for poorly water-soluble pharmaceutically active ingredients or for pharmaceutically active ingredients which show a high drug loss after storage. Preferably, the pharmaceutically active ingredient may be a drug poorly soluble, in particular water-soluble, after peroral administration.

The pharmaceutically active ingredient (ii) may show a solubility of less than 0.1 mg of pharmaceutically active ingredient, preferably of the pure pharmaceutically active ingredient, in 1 ml water at 37 °C (as defined for poorly insoluble drugs in the USP). The determination of the solubility of the pharmaceutically active ingredient is well known to a person skilled in the art. For instance, an excess amount of the pharmaceutically active ingredient is placed in a certain amount of water and mixed. The dissolved amount of the pharmaceutically active ingredient is then determined by a suitable analytical method, for instance by spectrometry.

In one embodiment the at least one pharmaceutically active ingredient may be selected from acalabrutinib, albendazole, allendronic acid, aripiprazole, asenapine, atazanavir, atorvastatin, BETd-260 bleomycin, bosentan, BRD4 degrader AT1, buprenorphine, budesonide, camostat, candesartan, carbamazepine, carvedilol, celecoxib, cilazapril, clarithromycin, clodronic acid, clopidogrel, curcumin, cytarabine, darunavir, dasatinib, deferasirox, dexamethasone, dexlansoprazole, diclofenac, diltiazem, docetaxel, doxorubicin, duloxetine, dutasteride, efavirenz, elbasvir, eprosartan, erlotinib, estradiol, etidronic acid, etravirine, everolimus, ezetimibe, felodipine, fenofibrate, fluconazole, fluorouracil, foretinib-based PROTAC 7, glimepiride, grazoprevir, griseovulvin, hydrochlorothiazide, hydrocortisone, hydroxychloroquine, ibuprofen, imatinib, irbesartan, irinotecan, itraconazole, ivacaftor, ivermectin, ledipasvir, lamotrigine, linezolid, lisinopril, lopinavir, losartan, lumefantrine, mefloquine, mesalazine, methotrexate, metoprolol, modafinil, moexipril, morphine, mycophenolate, naloxone, nifedipine, nilotinib, nilvadipine, nitrendipine, olanzapine, olmesartan, omeprazole, ondansetron, paclitaxel, pamidronic acid, paracetamol, pemetrexed, perindopril, phenytoin, pibrentasvir, pioglitazone, prednisone, progesterone, quetiapine, raloxifene, raltegravir, ramipril, rebamipide, remdesivir, rilpivirine, risedronic acid, risperidone, ritonavir, rivaroxaban, rivastigmine, rosuvastatin, selegiline, sevelamer, sibutramine, sildenafil, simvastatin, sirolimus, sitagliptin, sofosbuvir, sorafenib, spirapril, sunitinib, tacrolimus, tadalafil, tamoxifen, telaprevir, telmisartan, tenoxicam, terbutaline, ticagrelor, tiludronic acid, trandolapril, troglitazone, umifenovir, valsartan, velpatasvir, vemurafenib, verapamil, ziprasidone, zoledronic acid and ZXH-3-26, or, where applicable, from pharmaceutically acceptable salt forms thereof or mixtures thereof.

In another embodiment the at least one pharmaceutically active ingredient is selected from resveratrol from grape products or pro-anthocyanins or anthocyanins, in particular from bilberries or black currants, soluble dietary fiber products, such as psyllium seed, broccoli (sulphane), and soy or clover (isoflavonoids), flavonoids, alpha-linoleic acid from flaxseed, vitamin A, vitamin K2, coenzyme Q10, quercetin, trans-resveratrol, and beta-carotene from marigold petals.

Preferably, the at least one pharmaceutically active ingredient may be selected from celecoxib, efavirenz and fenofibrate or mixtures thereof.

### Lipid component (iii)

Any lipid component which can be used for pharmaceutical compositions is in general suitable.

The at least one lipid component (iii) may be selected from medium chain triglycerides (C₆-C₁₂ fatty acids), long chain triglycerides (C₁₃-C₂₁ fatty acids), propylene glycol dicaprylate / dicaprate (Captex® 200), glyceryl tricaprylate / tricaprate (Captex® 300), glyceryl triricinoleate (Castor oil), medium chain triglycerides (lauric acid) (Coconut oil), glyceryl dibehenate (Compritol® 888 ATO), triglycerides (linoleic acid, oleic acid) (Corn oil), triglycerides (linoleic acid, oleic acid, palmitic acid) (Cottonseed oil), ethyl oleate (Crodamol™ EO), glyceryl tricaprylate / tricaprate (Crodamol™ GTCC), isopropyl myristate (IPM-100), glyceryl tricaprylate / tricaprate (Labrafac™ CC), glyceryl tricaprylate / tricaprate (Labrafac™ lipophil WL 1349), propylene glycol dicaprylate / dicaprate (Labrafac™ PG), long chain triglycerides / diglycerides / monoglycerides (monolinoleate) (Maisine® CC), glyceryl tricaprylate / tricaprate / trilaurate (Miglyol® 812), oleic acid (Pamolyn™ 100 Oleic Acid), triglycerides (oleic acid, palmitic acid) (olive oil), triglycerides (palmitic acid, oleic acid, linoleic acid), triglycerides (oleic acid, linoleic acid, palmitic acid), triglycerides (linoleic acid, oleic acid, palmitic acid) (palm oil),triglycerides (linoleic acid, oleic acid, alpha-linolenic acid, palmitic acid) (sesame oil), triglycerides (linoleic acid, oleic acid, stearic acid) (soybean oil), glyceryl triacetate (Kollisolv® GTA), glyceryl tricaprylate (Tricaprylin®), hard fat (triglycerides / diglycerides) and hard fat (triglycerides) (Witepsol® H 35) or any mixture thereof.

Expressions in the above list with brackets indicate the main components of the lipid component (example: medium chain triglycerides (lauric acid)). Expressions in the above list with slashes indicate the components of the lipid component (example: glyceryl tricaprylate /tricaprate /trilaurate).

### Surfactant (iv)

Any surfactant which can be used for pharmaceutical compositions is in general suitable.

The at least one surfactant (iv), may comprise one or more surfactants and may be selected from polyoxyethylene (23) lauryl ether, polyoxyethylene (2) oleyl ether, glyceryl monooleate, medium chain monoglycerides / diglycerides (caprylate, caprate), glyceryl monocaprylate, propylene glycol monocaprylate, propylene glycol monocaprylate, polyoxyl-35 hydrogenated castor oil, polyoxyl-40 hydrogenated castor oil, lauroyl polyoxyl-32 glycerides, stearoyl polyoxyl-32 glycerides, polyoxyl-15 hydroxystearate, poloxamer 188 (triblock copolymer of polyoxyethylene and polyoxypropylene), poloxamer 407 (triblock copolymer of polyoxyethylene and polyoxypropylene), oleoyl polyoxyl-6 glycerides, linoleoyl polyoxyl-6 glycerides, lauroyl polyoxyl-6 glycerides, caprylocaproyl polyoxyl-8 glycerides, propylene glycol monolaurate (type II), propylene glycol monolaurate (type I), polyoxyl-40 stearate, diacetylated monoglyceride, glyceryl monooleate, polyglyceryl-3 dioleate, sorbitan monolaurate, sorbitan monooleate, sorbitan sesquioleate, sorbitan trioleate, glyceryl monostearate, d-alpha-tocopherol polyethylene glycol 1000 succinate (d-TPGS), polyoxyethylene sorbitan monolaurate, polyoxyethylene sorbitan monostearate and polyoxyethylene sorbitan monooleate or any mixture thereof.

### Solvent (v)

Any solvent which can be used for pharmaceutical compositions is in general suitable.

The at least one solvent (v) may be selected from diethylene glycol monoethyl ether, polyethylene glycol 200, polyethylene glycol 400, polyethylene glycol 6000, propane-1,2,3-triol, (z)-octadec-9-enylamine, polypropylene glycol, propylene glycol, 2-pyrrolidone and tetraethylene glycol or any mixture thereof.

In an embodiment the composition is essentially free of solvent.

### (Meth)acrylic copolymer (vi), different from (i)

The pharmaceutical composition of the present invention can further comprise at least one (meth)acrylic copolymer, different from (i). In general, all (meth)acrylic copolymers, which are suitable for pharmaceutical compositions can be used.

In one embodiment the at least one (meth)acrylic copolymer is a methacrylate copolymer obtained by polymerizing 40 to 60 wt.-% methacrylic acid and 60 to 40 wt.-% of ethyl acrylate, based on the total weight of the monomers.

Suitable polymers are for example commercially available under the tradename EUDRAGIT® from Evonik Nutrition & Care GmbH, Darmstadt, Germany.

In one embodiment the weight ratio of (i) to (vi) is of from 9:1 to 1:1, preferably from 4:1 to 2:1.

### Additive (vii)

Any additive which can be used for pharmaceutical compositions, different from (i) to (vi), is in general suitable.

Additives are preferably selected from antiadherents, like magnesium stearate; fillers, like lactose, mannitol, starches, cellulose and their derivatives; binders, like polyacrylates, starches, guar, xanthan, alginate, carrageenan, pectin, tragacanth, polysaccharides and their derivatives; flavors, like mint, cherry, anise, vanilla, raspberry; colors, like natural colorants, azo and xanthene compounds; pigments, like titanium dioxides, iron oxides, magnesium oxide; disintegrants, like starches, croscarmellose, crosslinked polyvinylpyrrolidone, sodium hydrogen carbonate preferably in combination with citric acid (for effervescent tablets); glidants, like silica gel, fumed silica, talc, magnesium carbonate; flow regulators, like highly dispersed silicon dioxide; antioxidants like vitamin A, vitamin E, vitamin C, retinyl palmitate, selenium, butylated hydroxyanisole, butylated hydroxytoluene; sweeteners, like sucrose, sorbitol, saccharin sodium, cyclamate, aspartame; and antistatics, like alkyl sulfonates or quaternary ammonium compounds preferably combined with polystyrene; or mixtures thereof.

### Self-nanoemulsifying drug delivery systems (SNEDDS) and solid self-nanoemulsifying drug delivery systems (S-SNEDDS)

A self-nanoemulsifying drug delivery system forms and remains a nanoemulsion in contact with water or gastrointestinal fluids. A skilled person in the field of nanoemulsions knows how to prepare the self-nanoemulsifying drug delivery system of the present invention with commonly known methods. The Z-Average size D_{z} (Z-Average particle size D_{z}) of the particles in the nanoemulsion may be between 1 and 1,000 nm, in many cases between 100 and 500 nm or from 10 to 100 nm. The nanoemulsion formation may happen during manufacturing of a pharmaceutical composition or by a medical professional or in vivo. The formation happens, when the emulsifying components and the pharmaceutically active ingredient are added to an aqueous media, preferably water. In one embodiment the formation is performed under stirring of the mixture and/or heating the mixture to 30 to 60 °C, preferably 45 to 55 °C, more preferably 50 °C. Stirring and/or heating can improve the provision of a homogenous mixture.

The emulsifying components of the self-nanoemulsifying drug delivery systems are usually comprising, or are consisting of, a lipid component, at least one surfactant and optionally a solvent, i.e. components (iii) to (iv). A skilled person in the field knows how to select the components and adjust the amounts of the components to the pharmaceutically active ingredient to be delivered. Thus, emulsifying components (iii) and (iv) or (iii) to (v) are mixed with a pharmaceutically active ingredient (ii) into a solution, which forms self-nanoemulsifying drug delivery system (SNEDDS).

The self-nanoemulsifying drug delivery system (SNEDDS) and a carrier, in the present invention the polymer(s) (i) and optionally (vi), form after processing, for instance by hot melt extrusion, spray drying, adsorption, electrospinning, electro spraying, prilling by vibration, granulation or supercritical fluid technology the solid self-nanoemulsifying drug delivery system (S-SNEDDS). In one preferred embodiment the S-SNEDDS are obtained by hot melt extrusion. If an additive (vii) is present, the additive is preferably compounded with the polymer(s) (i) and optionally (vi). In a preferred embodiment the polymers (i), and optionally (vi) as well as the optional additive (vii) are in powder form. More preferably, they have an average particle size D_{z} in the range of from 1 to 1,000 µm, most preferably from 100 to 500 µm. The powders can be obtained by conventional milling and grinding.

For example, the S-SNEDDS of the present invention are obtained by hot melt extrusion at a temperature of 140 to 180 °C, preferably of 145 to 170 °C. In one embodiment the components may be extruded in a twin-screw extruder. In one embodiment the components may be extruded in a twin-screw extruder at a torque of about 30 to 100 Ncm. Preferably, the components may be extruded in a twin-screw extruder at a torque of about 45 to 85 Ncm. The extruded mass may leave the extruder in the form of a strand, which may be comminuted by grinding and milling to a powder product.

In one embodiment the SNEDDS comprises or consists of
0.1 to 50 wt.-%, preferably 5 to 50 wt.-%, 10 to 25 wt.-%, of at least one pharmaceutically active ingredient (ii);
5 to 60 wt.-%, preferably 10 to 50 wt.-%, 25 to 35 wt.-% of at least one lipid component (iii);
10 to 60 wt.-%, preferably 10 to 50 wt.-%, 40 to 50 wt.-% of at least one surfactant (iv);
0 to 35 wt.-%, preferably 0.01 to 25 wt.-%, of at least one solvent (v), based on the total weight of the SNEDDS.

In one embodiment the S-SNEDDS comprise 1 to 50 wt.-%, preferably 1 to 40 wt.-%, more preferably 20 to 40 wt.-%, SNEDDS based on the total weight of the S-SNEDDS.

The solid self-nanoemulsifying drug delivery system (S-SNEDDS) may comprise a mixture, preferably a hot melt extruded mixture of
a) 20 to 40 wt.-% of a SNEDDS comprising or consisting of
   a1) 10 to 25 wt.-% of celecoxib as pharmaceutically active ingredient,
   a2) 25 to 35 wt.-% of medium chain triglycerides as lipid component,
   a3) 40 to 50 wt.-% of polysorbate 80 (polyoxyethylene(20)-sorbitan monooleate), 2 to 10 wt.-% of lauroyl polyoxyl-32 glycerides and 2 to 10 wt.-% of d-α-tocopherol polyethylene glycol 1000 succinate (d-TPGS) as surfactant component,
   wherein the components a1) to a3) may add up to 100 wt.-%, and
b) 80 to 60 wt.-% of dimethylaminopropyl methacrylamide-butyl methacrylate-methyl methacrylate copolymer. The components a) and b) of the mixture may add up to 100 wt.-%.

The Solid self-nanoemulsifying drug delivery system (S-SNEDDS) may comprise a mixture, preferably a hot melt extruded mixture of
a) 20 to 40 wt.-% of a SNEDDS comprising or consisting of:
   a1) 20 to 30 wt.-% of efavirenz as pharmaceutically active ingredient,
   a2) 15 to 25 wt.-% of isopropyl myristate as lipid component,
   a3) 20 to 30 wt.-% of polysorbate 80 (polyoxyethylene(20)-sorbitan monooleate) and 1 to 8 wt.-% of d-α-tocopherol polyethylene glycol 1000 succinate (d-TPGS) as surfactants, and
   a4) 25 to 35 wt.-% of diethylene glycol monoethyl ether as solvent,
   wherein the components a1) to a4) may add up to 100 wt.-%, and
b) 80 to 60 wt.-% of dimethylaminopropyl methacrylamide-butyl methacrylate-methyl methacrylate copolymer. The components a) and b) of the mixture may add up to 100 wt.-%.

The solid self-nanoemulsifying drug delivery system (S-SNEDDS) may comprise a mixture, preferably a hot melt extruded mixture of
a) 20 to 40 wt.-% of a SNEDDS comprising
   a1) 10 to 20 wt.-% of fenofibrate as pharmaceutically active ingredient,
   a2) 15 to 25 wt.-% of medium chain triglycerides as lipid component,
   a3) 5 to 15 wt.-% of polyoxyl-23 lauryl ether,
   45 to 55 wt.-% of polysorbate 80 (polyoxyethylene(20)-sorbitan monooleate) and
   5 to 15 wt.-% of d-α-tocopherol polyethylene glycol 1000 succinate (d-TPGS) as surfactants, wherein the components a1) to a3) of the solution may add up to 100% and
b) 80 to 60 wt.-% of dimethylaminopropyl methacrylamide-butyl methacrylate-methyl methacrylate copolymer. The components a) and b) of the mixture may add up to 100 wt.-%.

### Use as a medicament

The pharmaceutical compositions, including the solid self-nanoemulsifying drug delivery system (S-SNEDDS), of the present invention are suitable for use (method of use) as a medicament, which preferably enhances the solubility of the included pharmaceutically active ingredient compared to the pharmaceutically active ingredient alone in the treatment of a disease of a human or an animal subject.

The invention in particular pertains to:
1. Pharmaceutical composition, comprising or consisting of
   (i) at least one methacrylic copolymer comprising or consisting of units derived from methacrylamide, preferably alkyl methacrylamide or aminoalkyl methacrylamide,
      more preferably C₁₋₅ alkyl methacrylamide or dimethylamino C₁₋₅ alkyl methacrylamide or diethylamino C₁₋₅ alkyl methacrylamide,
      most preferably dimethylaminopropyl methacrylamide; and preferably alkyl methacrylate more preferably at least one C₁₋₅ alkyl methacrylate, most preferably methyl methacrylate and butyl methacrylate;
   (ii) at least one pharmaceutically active ingredient;
   (iii) at least one lipid component;
   (iv) at least one surfactant;
   (v) optionally at least one solvent;
   (vi) optionally at least one (meth)acrylic copolymer, different from (i); and
   (vii) optionally at least one additive.
2. Pharmaceutical composition according to item 1, wherein
   the at least one methacrylic copolymer comprising units derived from methacrylamide has
   i) an average weight molecular weight M_{w} of from 50,000 to 500,000 g/mol, preferably 100,000 to 295,000 g/mol, more preferably 150,000 to 260,000 g/mol; and/or
   ii) a glass transition temperature *T*_{g} of from 65 to 100 °C, preferably 70 to 85 or 82 °C.
3. Pharmaceutical composition according to item 1 or 2, wherein
   the at least one methacrylic copolymer comprising units derived from methacrylamide is a dimethylaminopropyl methacrylamide-butyl methacrylate-methyl methacrylate copolymer; preferably comprising at least 34 wt.-%, preferably 35 to 55 wt.-%, more preferably 45 to 50 wt.-%, of units derived from dimethylaminopropyl methacrylamide, based on the total weight of the copolymer.
4. Pharmaceutical composition according to any of the preceding items, wherein
   the at least one methacrylic copolymer comprising units derived from methacrylamide is obtained by radical polymerization, preferably by free radical polymerization, of 40 to 60 wt.-%, preferably 45 to 55 wt.-%, of dimethylaminopropyl methacrylamide, 15 to 35 wt.-%, preferably 20 to 30 wt.-%, of butyl methacrylate; and 15 to 35 wt.-%, preferably 20 to 30 wt.-%, of methyl methacrylate, wherein the sum of the monomers is 100 wt.-%, in the presence of at least one initiator, preferably selected from di-(3,5,5)trimethylhexanoyl peroxide, tert-butyl peroxyneodecanoate, tert-butyl perbenzoate, tert-amyl peroxy-2-ethylhexanoate, bisdecanoyl peroxide, tert-butyl peroxy-2-ethylhexanoate, tert-butyl peroxy-2-ethylhexylcabonate, 1,1'-azobis(cyclohexanecarbonitrile), benzoyl peroxide, 2,2-di-(tert-butylperoxy)butane, dicumyl peroxide, di-tert-amyl peroxide, di-tert-butyl peroxide, lauroyl peroxide, tert-butyl peroxy-3,5,5-trimethylhexanoate, 1,1-di-tert-butylperoxy-3,3,5-trimethylcyclohexane, 2-(1-cyan-1-methyl(ethyl)azocarboxamide, tert-butyl peroxyacetate, tert-butyl peroxypivalate or mixtures thereof, more preferably selected from tert-butyl peroxyneodecanoate and tert-butyl peroxypivalate or mixtures thereof and at least one chain-transfer agent preferably selected from carbon tetrachloride, carbon tetrabromide, bromotrichloromethane,
   4-methylbenzenethiol, isooctyl 3-mercaptopropionate, pentaphenylethane, tert-nonyl mercaptan, 4,4'-thiobisbenzenethiol and n-dodecyl mercaptan, more preferably which is n-dodecyl mercaptan, and optionally at least one solvent.
5. Pharmaceutical composition according to any of the preceding items, wherein the at least one pharmaceutically active ingredient has a solubility of less than 0.1 mg in 1 ml water at 37 °C and/or is selected from celecoxib, efavirenz and fenofibrate or mixtures thereof.
6. Pharmaceutical composition according to any of the preceding items, wherein the at least one lipid component is selected from medium chain triglycerides (C₆-C₁₂ fatty acids), long chain triglycerides (C₁₃-C₂₁ fatty acids), propylene glycol dicaprylate / dicaprate (Captex® 200), glyceryl tricaprylate / tricaprate (Captex® 300), glyceryl triricinoleate (Castor oil), medium chain triglycerides (lauric acid) (Coconut oil), glyceryl dibehenate (Compritol® 888 ATO), triglycerides (linoleic acid, oleic acid) (Corn oil), triglycerides (linoleic acid, oleic acid, palmitic acid) (Cottonseed oil), ethyl oleate (Crodamol™ EO), glyceryl tricaprylate / tricaprate (Crodamol™ GTCC), isopropyl myristate (IPM-100), glyceryl tricaprylate / tricaprate (Labrafac™ CC), glyceryl tricaprylate / tricaprate (Labrafac™ lipophil WL 1349), propylene glycol dicaprylate / dicaprate (Labrafac™ PG), long chain triglycerides / diglycerides / monoglycerides (monolinoleate) (Maisine® CC), glyceryl tricaprylate / tricaprate /trilaurate (Miglyol® 812), oleic acid (Pamolyn™ 100 Oleic Acid), triglycerides (oleic acid, palmitic acid) (olive oil), triglycerides (palmitic acid, oleic acid, linoleic acid), triglycerides (oleic acid, linoleic acid, palmitic acid), triglycerides (linoleic acid, oleic acid, palmitic acid) (palm oil),triglycerides (linoleic acid, oleic acid, alpha-linolenic acid, palmitic acid) (sesame oil), triglycerides (linoleic acid, oleic acid, stearic acid) (soybean oil), glyceryl triacetate (Kollisolv® GTA), glyceryl tricaprylate (Tricaprylin®), hard fat (triglycerides / diglycerides) and hard fat (triglycerides) (Witepsol® H 35) or any mixtures thereof.
7. Pharmaceutical composition according to any of the preceding items, wherein the at least one surfactant is selected from polyoxyethylene-(23) lauryl ether (Brij® 35), polyoxyethylene-(2) oleyl ether (Brij® 92), glyceryl monooleate (Capmul® GMO-50), medium chain monoglycerides / diglycerides (caprylate, caprate) (Capmul® MCM), glyceryl monocaprylate (Capmul® MCM C-8), propylene glycol monocaprylate (Capmul® PG-8), propylene glycol monocaprylate (Capryol™ 90), polyoxyl-35 hydrogenated castor oil (Cremophor® EL, Kolliphor® EL) polyoxyl-40 hydrogenated castor oil (Cremophor® RH 40, Kolliphor® RH 40), lauroyl polyoxyl-32 glycerides (Gelucire® 44/14), stearoyl polyoxyl-32 glycerides (Gelucire® 50/13), polyoxyl-15 hydroxystearate (Kolliphor® HS 15, Solutol® HS 15), poloxamer 188 (triblock copolymer of polyoxyethylene and polyoxypropylene) (Kolliphor® P 188, Lutrol® F 68, Pluronic® F 68), poloxamer 407 (triblock copolymer of polyoxyethylene and polyoxypropylene) (Kolliphor® P 407, Lutrol® F 127, Pluronic® F 127), oleoyl polyoxyl-6 glycerides (Labrafil® M 1944 CS), linoleoyl polyoxyl-6 glycerides (Labrafil® M 2125 CS), lauroyl polyoxyl-6 glycerides (Labrafil® M 2130 CS), caprylocaproyl polyoxyl-8 glycerides (Labrasol®), propylene glycol monolaurate (type II) (Lauroglycol™ 90), propylene glycol monolaurate (type I) (Lauroglycol™ FCC), polyoxyl-40 stearate (Myrj™ S40), diacetylated monoglyceride (Myvacet™ 9-45 K NF), glyceryl monooleate (Peceol™), polyglyceryl-3 dioleate (Plural® Oleique CC 497), sorbitan monolaurate (Span® 20), sorbitan monooleate (Span® 80), sorbitan sesquioleate (Span® 83), sorbitan trioleate (Span® 85), glyceryl monostearate (SustOleo™ GMS-SE), d-α-tocopherol polyethylene glycol 1000 succinate (d-TPGS) (Tocophersolan®), polyoxyethylene sorbitan monolaurate (Tween® 20), polyoxyethylene sorbitan monostearate (Tween® 60) and polyoxyethylene sorbitan monooleate (Tween® 80) or any mixtures thereof.
8. Pharmaceutical composition according to any of the preceding items, wherein the at least one solvent is selected from diethylene glycol monoethyl ether (Carbitol™), polyethylene glycol 200, polyethylene glycol 400, polyethylene glycol 6000, propane-1,2,3-triol (glycerine), (z)-octadec-9-enylamine (oleylamine), polypropylene glycol, propylene glycol, 2-pyrrolidone (Soluphor® P), tetraethylene glycol and diethylene glycol monoethyl ether (Transcutol® HP) or any mixtures thereof.
9. Pharmaceutical composition according to any of the preceding items, wherein the at least one (meth)acrylic copolymer, different from (i), is a methacrylate copolymer obtained by polymerizing 40 to 60 wt.-% methacrylic acid and 60 to 40 wt.-% of ethyl methacrylate, based on the total weight of the monomers.
10. Pharmaceutical composition according to any of the preceding items, wherein the at least one additive is selected from antiadherents, like magnesium stearate; fillers, like lactose, mannitol, starches, cellulose and their derivatives; binders, like polyacrylates, starches, guar, xanthan, alginate, carrageenan, pectin, tragacanth, polysaccharides and their derivatives; flavors, like mint, cherry, anise, vanilla, raspberry; colors, like natural colorants, azo and xanthene compounds; pigments, like titanium dioxides, iron oxides, magnesium oxide; disintegrants, like starches, croscarmellose, crosslinked polyvinylpyrrolidone, sodium hydrogen carbonate preferably in combination with citric acid (for effervescent tablets); glidants, like silica gel, fumed silica, talc, magnesium carbonate; flow regulators, like highly dispersed silicon dioxide; antioxidants, like vitamin A, vitamin E, vitamin C, retinyl palmitate, selenium, butylated hydroxyanisole, butylated hydroxytoluene; sweeteners, like sucrose, sorbitol, saccharin sodium, cyclamate, aspartame; and antistatics, like alkyl sulfonates or quaternary ammonium compounds preferably combined with polystyrene; or mixtures thereof.
11. Pharmaceutical composition according to any of the preceding items, wherein
   (i) is present in 40 to 99 wt.-%, preferably 80 to 99 wt.-%, more preferably 90 to 99 wt.-%, based on the total weight of the composition; and
   (ii) to (vii) are present in 1 to 60 wt.-%, preferably 1 to 20 wt.-%, more preferably 1 to 10 wt.-%, based on the total weight of the composition, wherein
      (ii) is present in 0.1 to 50 wt.-%, preferably 0.5 to 20 wt.-%, more preferably 1 to 10 wt.-%;
      (iii) is present in 5 to 60 wt.-%, preferably 10 to 45 wt.-%, more preferably 15 to 35 wt.-%;
      (iv) is present in 1 to 60 wt.-%, preferably 3 to 55 wt.-%, more preferably 5 to 50 wt.-%;
      (v) is present in 0 to 35 wt.-%, preferably 0.01 to 32 wt.-%, more preferably 15 to 30 wt.-%;
      (vi) is present in 0 to 84 wt.-%, preferably 5 to 50 wt.-%, more preferably 10 to 40 wt.-%;
      (vii) is present in 0 to 10 wt.-%, preferably 0.01 to 5 wt.-%, more preferably 0.1 to 2 wt.-%; based on the total weight of components (ii) to (vii) respectively,
      preferably the sum of components (i) to (vii) is 100 wt.-%, based on the total weight of the composition.
12. Pharmaceutical composition according to any of the preceding items, wherein the pharmaceutical composition is a solid self-nanoemulsifying drug delivery system, preferably obtained by hot melt extrusion, spray drying, adsorption, electrospinning, electro spraying, prilling by vibration, granulation or supercritical fluid technology.
13. The pharmaceutical composition according to any of items 1 to 12 for use as a medicament, wherein preferably the pharmaceutical composition enhances the solubility of the included pharmaceutically active ingredient compared to the pharmaceutically active ingredient alone in the treatment of a disease of a human or an animal subject.
14. Method of preparing a solid self-nanoemulsifying drug delivery system comprising or consisting of the steps:
   providing a self-nanoemulsifying drug delivery system by mixing compounds (ii) to (iv) and optionally (v) of any of items 1 to 11, and then
   applying the obtained self-nanoemulsifying drug delivery system on compound (i) and optionally compounds (vi) and (vii), preferably in powder form, of any of items 1 to 11 by hot melt extrusion, spray drying, adsorption, electrospinning, electrospraying, prilling by vibration, granulation or supercritical fluidization, preferably by hot melt extrusion, more preferably by hot melt extrusion at a temperature of 140 to 180 °C, preferably of 145 to 170 °C, to obtain a solid self-nanoemulsifying drug delivery system.
15. Method according to item 14, wherein hot melt extrusion is performed in a twin-screw extruder, preferably at a torque of 30 to 100 Ncm, more preferably 45 to 85 Ncm.
16. Solid self-nanoemulsifying drug delivery system obtained by the method of item 14 or 15.

### Examples

### Materials & Methods

### Materials

Celecoxib (4-[5-(4-methylphenyl)-3-(trifluoromethyl)pyrazol-1-yl]benzenesulfonamide) was used as a model compound and obtained from Aarti Drugs Ltd. (Mumbai, India). Efavirenz ((4S)-6-chloro-4-(2-cyclopropylethynyl)-4-(trifluoromethyl)-1H-3,1-benzoxazin-2-one) was obtained from Angene International Ltd. (London, United Kingdom) and fenofibrate (propan-2-yl 2-[4-(4-chlorobenzoyl)phenoxy]-2-methylpropanoate) from D.K. Pharma Chem PVT Ltd. (Maharashtra, India). Polyoxyethylene (80) sorbitan monooleate (Tween® 80), d-α-Tocopherol polyethylene glycol 1000 succinate (d-TPGS), isopropyl myristate (IPM-100) and polyoxyethylene (23) lauryl ether (Brij® 35) were purchased from Sigma Aldrich (Steinheim, Germany). Lauroyl polyoxyl-32 glycerides (Gelucire® 44/14) and diethylene glycol monoethyl ether (Transcutol® HP) were kindly donated by Gattefosse S.A.S (Saint Priest, France). Medium-chain triglycerides (Miglyol® 812) was obtained from Caesar & Loretz GmbH (Hilden, Germany). Dimethylaminopropyl methacrylamide-butyl methacrylate-methyl methacrylate copolymers (monomer weight ratio 2:1:1) (E-173 kDa, E-254 kDa, E-281 kDa, E-305 kDa) are products of research studies from Evonik Nutrition & Care GmbH (Darmstadt, Germany). Polyvinyl caprolactam-polyvinyl acetate-polyethylene glycol graft copolymer (Soluplus®), polyvinylpyrrolidone-polyvinyl acetate copolymer (Kollidon® VA 64) and polyvinylpyrrolidone (Kollidon® 17 PF) were purchased from BASF SE (Ludwigshafen, Germany). Hydroxypropyl methylcellulose acetate succinate (AQOAT® AS-MMP) was kindly donated by Shin-Etsu Chemical Co.,Ltd. (Tokio, Japan). Hydroxypropyl methylcellulose (Affinisol® HPMC 100 LV) was provided by Dow Chemical Company (Schwalbach am Taunus, Germany). All other chemicals and solvents were of analytical grade and purchased commercially.

### Methods

### Preparation of the dimethylaminopropyl methacrylamide-butyl methacrylate-methyl methacrylate copolymer (2:1:1 (ratios by weight))

For the preparation of the dimethylaminopropyl methacrylamide-butyl methacrylate-methyl methacrylate copolymers, the monomers dimethylaminopropyl methacrylamide (500.0 g), butyl methacrylate (250.0 g) and methyl methacrylate (250.0 g) were added into a 3,000 ml round-bottom flask equipped with a magnetic bar, a return condenser and a nitrogen inlet. The reaction vessel was placed in a preheated water bath at 82 °C. When reaching an internal temperature of 65 °C, n-dodecyl mercaptan (3.0 g, 5.0 g, 9.0 g or 15.0 g) was added immediately to control the molecular weight of the polymer during the process, while a solution of tert-butyl peroxyneodecanoate (6.0 g) and n-propanol (1,000 g) was added with a continuous flow rate of 5 g/min to initiate a free radical polymerization. After approximately 3 h at a temperature between 80 - 85 °C, the second initiator, the tert-butyl peroxypivalate (0.5 g) completed the reaction within another 90 min. The mixtures were cooled down and transferred to an oven at 40 °C in order to remove n-propanol over 48 h. The polymers were purified using deionized water and were dried afterwards over 10 days at 50 °C. The dry, coarse polymers were ground (mesh size: 0.25 mm) using an Ultra Centrifugal Mill ZM 200 from Retsch GmbH (Haan, Germany).

Obtained were four copolymers having different weight average molecular weights of 173 kDa, 254 kDa, 281 kDa, and 305 kDa, referred to as polymers E-173 kDa, E-254 kDa, E-281 kDA, and E-305 kDA in the following.

### Residual monomer (ReMo) analysis & monomer conversion rate

The residual monomers of the dimethylaminopropyl methacrylamide-butyl methacrylate-methyl methacrylate copolymers were analyzed before drying using HPLC analysis regarding dimethylaminopropyl methacrylamide as well as GC analysis referring to butyl methacrylate and methyl methacrylate. Based on the results of the residual monomer analysis (6.16 wt.-% for dimethylaminopropyl methacrylamide, 0.002 wt.-% for butyl methacrylate and 0.035 wt.-% for methyl methacrylate), considering a monomer ratio of 2:1:1 (dimethylaminopropyl methacrylamide: butyl methacrylate: methyl methacrylate), the average monomer conversion rate was calculated at 87.68 % for dimethylaminopropyl methacrylamide, 99.99 % for butyl methacrylate and 99.86 % for methyl methacrylate. That lead to a final polymer composition of 46.74 wt.-% dimethylaminopropyl methacrylamide, 26.65 wt.-% butyl methacrylate and 26.61 wt.-% methyl methacrylate.

### Gel permeation chromatography (GPC)

The molecular weight distribution of dimethylaminopropyl methacrylamide-butyl methacrylate-methyl methacrylate copolymer was determined using an Agilent 1100 Series GPC-SEC Analysis System comprising a pump (G1310A), autosampler (G1313A), column oven (G1316A), RI-detector (G1362A) and a control module (G1323B) obtained from Agilent Technologies (Frankfurt am Main, Germany). Separation was achieved using a GRAM precolumn (8 x 50 mm, 10 µm) and another three GRAM columns (8 x 300 mm, 10 µm), all maintained at 60 °C. The eluent consisted of n,n-dimethylacetamide: lithium bromide: tris(hydroxymethyl)aminomethane (TRIS): water (1000:2:2:10 w/w), the flow rate was set to 1 ml/min and an injection volume of 100 µl was applied. The RI-detector was maintained at 40 °C and a polymethylmethacrylate solution (1 g/l) was used as a standard. The number-average molecular weight (Mₙ), the weight-average molecular weight (M_{w}) and the polydispersity index (PDI) of dimethylaminopropyl methacrylamide-butyl methacrylate-methyl methacrylate copolymer were determined (see table 12).

### Preparation of S-SNEDDS via hot-melt extrusion

A blend in a stoichiometric ratio of the compounds 1 to 4 as well as the specific pharmaceutically active ingredient, in the following referred to as drug as well, (Tables 1 to 3) was prepared by mixing these substances in a beaker glass under moderate magnetic stirring for approximately 30 min. The blend was subjected to a temperature of 50 °C while stirring. The obtained SNEDDS-solution was added to a certain polymer (Tables 4, 6, and 8) considering a defined mixture ratio. The solution/polymer blend was processed via hot-melt extrusion technology using a co-rotating HAAKE MiniLab twin screw extruder from Thermo Fisher Scientific (Dreieich, Germany) that exhibited a conical screw design. The hot-melt extrusion process was characterized by recording the applied screw speed, the torque and the process temperature (Tables 4, 6, and 8). The continuously generated strand, leaving the extruder at its nozzle, cooled down while it was transported using a conveying belt and was finally chopped into a coarse granule. The granule was ground (mesh size: 0.25 mm) using an Ultra Centrifugal Mill ZM 200 from Retsch GmbH (Haan, Germany). The obtained powder was the dosage form of the manufactured S-SNEDDS that was used for all further studies. In addition, a blend of polymer and drug substance (PODS), (Tables 5, 7, and 9) was processed via the mentioned hot-melt extrusion process in order to assess the effect of SNEDDS referring to different characteristics in comparison to PODS.

### Dissolution studies of drug substances, PODS & Solid-SNEDDS

### Dissolution experiments were performed according to USP 42-NF 37 (2019).

Dissolution experiments were performed with 25 mg drug substance or an equivalent amount of PODS or Solid-SNEDDS using USP apparatus II ((DT 800 LH) from ERWEKA GmbH Heusenstamm, Germany). The paddle speed was set to 100 rpm and all experiments were performed in 500 ml of 0.1N hydrochloric acid. The dissolution tests were conducted over 120 min.

### HPLC method for analyzing celecoxib

The high-performance liquid chromatography (HPLC) system (Agilent 1260 Infinity) used for the quantification of celecoxib consisted of a quaternary pump (G1311B), autosampler (G1329B), column oven (G1316A) and UV detector (G1314C), all obtained from Agilent Technologies (Frankfurt am Main, Germany). Separation was achieved using a Knauer Nucleosil 100-7 C18 (125 x 4.6 mm, 7 µm) column maintained at 40 °C. The mobile phase consisted of an acetonitrile: water: triethylamine mixture (300:300:0.9 v/v), adjusted to pH 3.00 with phosphoric acid. The flow rate was set to 1.8 ml/min. An injection volume of 5 µl was applied and celecoxib was detected at 254 nm. In the concentration range of 0.13 - 542 µg/ml, the analytical curve was linear (r² = 0.999995). The method was found to be accurate (100.2 - 102.1%) and precise (CV 2.46%) with a quantification limit of 0.05 µg/ml. Run time was defined to be 7 min. Selectivity was determined (formulation excipients) and no interference was observed in drug retention time. Moreover, the peak area did not change in the presence of all excipient used in the study.

### HPLC method for analyzing efavirenz

The high-performance liquid chromatography (HPLC) system (Agilent 1260 Infinity) used for the quantification of efavirenz consisted of a quaternary pump (G1311 B), autosampler (G1329B), column oven (G1316A) and UV detector (G1314C), all obtained from Agilent Technologies (Frankfurt am Main, Germany). Separation was achieved using a Symmetry 300 C18 (250 x 4.6 mm, 5 µm) column maintained at 22 °C. The mobile phase consisted of an acetonitrile: buffer solution (disodium hydrogen phosphate/phosphoric acid adjusted to pH 3.60) mixture (290:210 v/v). The flow rate was set to 1.5 ml/min. An injection volume of 20 µl was applied and efavirenz was detected at 247 nm. In the concentration range of 0.13 - 515 µg/ml, the analytical curve was linear (r² = 0.999894). The method was found to be accurate (101.4 - 103.0%) and precise (CV 4.05%) with a quantification limit of 0.05 µg/ml. Run time was defined to be 10 min. Selectivity was determined (formulation excipients) and no interference was observed in drug retention time. Moreover, the peak area did not change in the presence of all excipient used in the study.

### HPLC method for analyzing fenofibrate

The high-performance liquid chromatography (HPLC) system (Agilent 1260 Infinity) used for the quantification of fenofibrate consisted of a quaternary pump (G1311B), autosampler (G1329B), column oven (G1316A) and UV detector (G1314C), all obtained from Agilent Technologies (Frankfurt am Main, Germany). Separation was achieved using a Symmetry 300 C18 (150 x 4.6 mm, 5 µm) column maintained at 22 °C. The mobile phase consisted of an acetonitrile: water mixture (70:30 v/v), adjusted to pH 2.50 with phosphoric acid. The flow rate was set to 2.0 ml/min. An injection volume of 20 µl was applied and fenofibrate was detected at 286 nm. In the concentration range of 0.13 - 526 µg/ml, the analytical curve was linear (r² = 0.999992). The method was found to be accurate (101.2 - 101.4%) and precise (CV 2.42%) with a quantification limit of 0.05 µg/ml. Run time was defined to be 6 min. Selectivity was determined (formulation excipients) and no interference was observed in drug retention time. Moreover, the peak area did not change in the presence of all excipient used in the study.

### Differential scanning calorimetry (DSC) analysis (DIN EN ISO 11357-2:2013)

Solid-SNEDDS and PODS were thermally analyzed via DSC to determine if the incorporated drug demonstrated an amorphous (glass transition) or crystalline (melting/crystallization peak) appearance. The glass transition is a reversible transition from a hard and relatively brittle, frozen state to a molten or rather rubbery state within amorphous or partly amorphous materials. The melting point of the pure drug substance as well as the glass transition temperature of the polymer were investigated for identifying changes and/or shifts in the thermograms of Solid-SNEDDS and PODS regarding crystalline and/or amorphous characteristics. A sample of 5 - 10 mg each was weighed into a small, perforated aluminum pan with a lid that was cold sealed and exposed to a heating-cooling-heating cycle starting from 0 °C up to 200 °C while running the measurement continuously applying inert nitrogen atmosphere. The constant heating/cooling rate was set to 10 °C/min. In the resulting thermogram the heat flow is plotted against the temperature using an endothermic presentation method. The evaluation was based on the second heating cycle, and the indicated value is the mean value in the glass transition interval. The analysis was conducted using a DSC 3+ (DSC-HC01) from Mettler Toledo (Gießen, Germany).

### Stability studies

Solid-SNEDDS and PODS were stored at constant and controlled conditions (30 °C/65% RH) in a climatic chamber from Binder GmbH (Tuttlingen, Germany) over 6 months. The samples were kept in a 30 ml amber glass, closed with a screw cap. After 3 and 6 months, samples were withdrawn and the results regarding appearance, drug release and DSC were compared to the data of the samples at the time of manufacture.

### Composition of SNEDDS-solutions

**Table 1: Composition of SNEDDS-solution incorporating celecoxib**

| | **compound 1** | **compound 2** | **compound 3** | **compound 4** | **drug substance** |
|---|---|---|---|---|---|
| **trade name** | Miglyol® 812 | Tween® 80 | Gelucire® 44/14 | d-TPGS | celecoxib |
| **amount [%]** | 27.64 | 45.52 | 4.15 | 5.69 | 17.00 |

**Table 2: Composition of SNEDDS-solution incorporating efavirenz**

| | **compound 1** | **compound 2** | **compound 3** | **compound 4** | **drug substance** |
|---|---|---|---|---|---|
| **trade name** | IPM-100 | Tween® 80 | Transcutol® HP | d-TPGS | efavirenz |
| **amount [%]** | 19.45 | 23.94 | 28.42 | 2.99 | 25.20 |

**Table 3: Composition of SNEDDS-solution incorporating fenofibrate**

| | **compound 1** | **compound 2** | **compound 3** | **compound 4** | **drug substance** |
|---|---|---|---|---|---|
| **trade name** | Miglyol® 812 | Brij® 35 | Tween® 80 | d-TPGS | fenofibrate |
| **amount [%]** | 17.20 | 8.60 | 50.16 | 10.04 | 14.00 |

### Composition & hot-melt extrusion process parameters of Solid-SNEDDS and PODS

All evaluated polymers were processed with a SNEDDS solution load of 30 % via hot-melt extrusion with the exception of Soluplus®, which was only processable up to a SNEDDS solution load of 20 %. Soluplus® exhibited the lowest SNEDDS solution load and therefore the lowest drug load of all Solid-SNEDDS formulations.

**Table 4: Composition & hot-melt extrusion process parameters of Solid-SNEDDS incorporating celecoxib (composition of table 1)**

| **polymer** | **SNEDDS-solution loading [%]** | **total drug load [%]** | **extrusion temperature [°C]** | **torque [Ncm]** | **screw speed [rpm]** |
|---|---|---|---|---|---|
| Soluplus® | 20 | 3.4 | 130 | 50 | 200 |
| Kollidon® VA 64 | 30 | 5.1 | 150 | 45 | 200 |
| Kollidon® 17 PF | 30 | 5.1 | 180 | 40 | 200 |
| AQOAT® AS-MMP | 30 | 5.1 | 150 | 40 | 200 |
| E-173 kDa | 30 | 5.1 | 150 | 75 | 200 |
| E-254 kDa | 30 | 5.1 | 150 | 75 | 200 |
| E-281 kDa | 30 | 5.1 | 155 | 70 | 200 |
| E-305 kDa | 30 | 5.1 | 155 | 60 | 200 |
| Affinisol® HPMC 100 LV | 30 | 5.1 | 160 | 85 | 100 |

**Table 5: Composition & hot-melt extrusion process parameters of PODS incorporating celecoxib**

| **polymer** | **total drug load [%]** | **extrusion temperature [°C]** | **torque [Ncm]** | **screw [rpm]** | **speed** |
|---|---|---|---|---|---|
| Soluplus® | 3.4 | 150 | 130 | 200 | |
| Kollidon® VA 64 | 5.1 | 160 | 70 | 200 | |
| Kollidon® 17 PF | 5.1 | 195 | 55 | 200 | |
| AQOAT® AS-MMP | 5.1 | 170 | 140 | 200 | |
| E-173 kDa | 5.1 | 150 | 200 | 200 | |
| E-254 kDa | 5.1 | 165 | 190 | 200 | |
| E-281 kDa | 5.1 | 160 | 200 | 200 | |
| E-305 kDa | 5.1 | 165 | 230 | 200 | |
| Affinisol® HPMC 100 LV | 5.1 | 165 | 150 | 100 | |

**Table 6: Composition & hot-melt extrusion process parameters of Solid-SNEDDS incorporating efavirenz (composition of table 2)**

| **polymer** | **SNEDDS-solution loading [%]** | **total drug load [%]** | **extrusion temperature [°C]** | **torque [Ncm]** | **screw speed [rpm]** |
|---|---|---|---|---|---|
| Soluplus® | 16.67 | 4.2 | 130 | 40 | 200 |
| Kollidon® VA 64 | 25 | 6.3 | 150 | 45 | 200 |
| Kollidon® 17 PF | 25 | 6.3 | 170 | 40 | 200 |
| AQOAT® AS-MMP | 25 | 6.3 | 150 | 50 | 200 |
| E-173 kDa | 25 | 6.3 | 150 | 65 | 200 |
| E-254 kDa | 25 | 6.3 | 150 | 70 | 200 |
| E-281 kDa | 25 | 6.3 | 155 | 65 | 200 |
| E-305 kDa | 25 | 6.3 | 155 | 90 | 200 |
| Affinisol® HPMC 100 LV | 25 | 6.3 | 160 | 120 | 100 |

**Table 7: Composition & hot-melt extrusion process parameters of PODS incorporating efavirenz**

| **polymer** | **total drug load [%]** | **extrusion temperature [°C]** | **torque [Ncm]** | **screw [rpm]** | **speed** |
|---|---|---|---|---|---|
| Soluplus® | 4.2 | 150 | 105 | 200 | |
| Kollidon® VA 64 | 6.3 | 165 | 90 | 200 | |
| Kollidon® 17 PF | 6.3 | 190 | 85 | 200 | |
| AQOAT® AS-MMP | 6.3 | 175 | 200 | 200 | |
| E-173 kDa | 6.3 | 160 | 185 | 200 | |
| E-254 kDa | 6.3 | 160 | 180 | 200 | |
| E-281 kDa | 6.3 | 160 | 200 | 200 | |
| E-305 kDa | 6.3 | 165 | 200 | 200 | |
| Affinisol® HPMC 100 LV | 6.3 | 165 | 150 | 100 | |

**Table 8: Composition & hot-melt extrusion process parameters of Solid-SNEDDS incorporating fenofibrate (composition of table 3)**

| **polymer** | **SNEDDS-solution loading [%]** | **total drug load [%]** | **extrusion temperature [°C]** | **torque [Ncm]** | **screw speed [rpm]** |
|---|---|---|---|---|---|
| Soluplus® | 20 | 2.8 | 130 | 45 | 200 |
| Kollidon® VA 64 | 30 | 4.2 | 150 | 35 | 200 |
| Kollidon® 17 PF | 30 | 4.2 | 175 | 35 | 200 |
| AQOAT® AS-MMP | 30 | 4.2 | 150 | 60 | 200 |
| E-173 kDa | 30 | 4.2 | 150 | 50 | 200 |
| E-254 kDa | 30 | 4.2 | 155 | 70 | 200 |
| E-281 kDa | 30 | 4.2 | 150 | 75 | 200 |
| E-305 kDa | 30 | 4.2 | 155 | 85 | 200 |
| Affinisol® HPMC 100 LV | 30 | 4.2 | 160 | 95 | 100 |

**Table 9: Composition & hot-melt extrusion process parameters of PODS incorporating fenofibrate**

| **polymer** | **total drug load [%]** | **extrusion temperature [°C]** | **torque [Ncm]** | **screw [rpm]** | **speed** |
|---|---|---|---|---|---|
| Soluplus® | 2.8 | 150 | 95 | 200 | |
| Kollidon® VA 64 | 4.2 | 170 | 65 | 200 | |
| Kollidon® 17 PF | 4.2 | 190 | 65 | 200 | |
| AQOAT® AS-MMP | 4.2 | 175 | 140 | 200 | |
| E-173 kDa | 4.2 | 160 | 180 | 200 | |
| E-254 kDa | 4.2 | 165 | 180 | 200 | |
| E-281 kDa | 4.2 | 160 | 200 | 200 | |
| E-305 kDa | 4.2 | 165 | 220 | 200 | |
| Affinisol® HPMC 100 LV | 4.2 | 170 | 120 | 100 | |

### Thermal characterization of the drug, pure polymer, PODS & Solid-SNEDDS via DSC analysis

**Table 10: Melting points of crystalline drug substances by DSC analysis**

| | **celecoxib** | **efavirenz** | **fenofibrate** |
|---|---|---|---|
| **melting point [°C]** | 158 | 140 | 82 |

**Table 11: Glass transition temperature (T_{g}) of pure polymer, PODS & Solid-SNEDDS**

| **polymer** | ***T*_{g} (polymer) [°C]** | ***T*_{g} (PODS-celecoxib) [°C]** | ***T*_{g} (Solid-SNEDDS-celecoxib) [°C]** | ***T*_{g} (PODS-efavirenz) [°C]** | ***T*_{g} (Solid-SNEDDS-efavirenz) [°C]** | ***T*_{g} (PODS-fenofibrate) [°C]** | ***T*_{g} (Solid-SNEDDS-fenofibrate) [°C]** |
|---|---|---|---|---|---|---|---|
| Soluplus® | 70 | 67 | 39 | 69 | 42 | 67 | 31 |
| Kollidon® VA 64 | 107 | 100 | 67 | 100 | 43 | 92 | 47 |
| Kollidon® 17 PF | 136 | 126 | 88 | 96 | 60 | 90 | 74 |
| AQOAT® AS-MMP | 113 | 106 | 55 | 100 | 44 | 101 | 47 |
| E-173 kDa | 77 | 77 | 43 | 78 | 38 | 74 | 48 |
| E-254 kDa | 85 | 79 | 44 | 78 | 37 | 77 | 41 |
| E-281 kDa | 89 | 84 | 45 | 83 | 38 | 76 | 48 |
| E-305 kDa | 91 | 84 | 42 | 82 | 42 | 80 | 44 |
| Affinisol® HPMC 100 LV | 103 | 90 | 45 | 87 | 43 | 84 | 36 |

All samples (Table 11) were analyzed immediately after processing via the mentioned DSC method and showed an amorphous appearance (no crystallization peaks). The *T*_{g} of Solid-SNEDDS and PODS is usually lower in comparison to the pure polymer.

### Residual monomer (ReMo) analysis & monomer conversion rate

Based on the results (average of Table 12) of the residual monomer analysis (6.16 wt.-% for dimethylaminopropyl methacrylamide, 0.002 wt.-% for butyl methacrylate and 0.035 wt.-% for methyl methacrylate) considering a monomer ratio of 2:1:1 (dimethylaminopropyl methacrylamide: butyl methacrylate: methyl methacrylate), the average monomer conversion rate was calculated at 87.68 % for dimethylaminopropyl methacrylamide, 99.99 % for butyl methacrylate and 99.86 % for methyl methacrylate. That leads to a final polymer composition of 46.74 wt.-% dimethylaminopropyl methacrylamide, 26.65 wt.-% for butyl methacrylate and 26.61 wt.-% for methyl methacrylate.

**Table 12: Residual monomer content, monomer conversion rate and the final polymer composition of dimethylaminopropyl methacrylamide-butyl methacrylate-methyl methacrylate copolymer**

| **polymer** | **residual monomers (ReMo)** | | | **monomer conversion rate** | | | **final polymer composition** | | |
|---|---|---|---|---|---|---|---|---|---|
| | DMAPMA* [%] | BMA* [%] | MMA* [%] | DMAPMA* [%] | BMA* [%] | MMA* [%] | DMAPMA* [%] | BMA* [%] | MMA* [%] |
| **E-173 kDa** | 6.48 | 0.002 | 0.022 | 87.04 | 99.99 | 99.91 | 46.55 | 26.74 | 26.71 |
| **E-254 kDa** | 6.79 | 0.002 | 0.037 | 86.42 | 99.99 | 99.85 | 46.38 | 26.83 | 26.79 |
| **E-281 kDa** | 5.59 | 0.002 | 0.034 | 88.82 | 99.99 | 99.86 | 47.06 | 26.49 | 26.45 |
| **E-305 kDa** | 5.78 | 0.002 | 0.045 | 88.44 | 99.99 | 99.82 | 46.96 | 26.54 | 26.50 |
| **average** | 6.16 | 0.002 | 0.035 | 87.68 | 99.99 | 99.86 | 46.74 | 26.65 | 26.61 |

| | | | | | | | | | |
|---|---|---|---|---|---|---|---|---|---|
| *DMAPMA = dimethylaminopropyl methacrylamide, BMA = butyl methacrylate, MMA = methyl methacrylate | | | | | | | | | |

### Gel permeation chromatography (GPC)

Running the described GPC-method, the number-average molecular weight (Mₙ), the weight-average molecular weight (M_{w}) and the polydispersity index (PDI) were determined for the dimethylaminopropyl methacrylamide-butyl methacrylate-methyl methacrylate copolymer that was synthesized using four different concentrations of the chain-transfer-agent n-dodecyl mercaptan. The results are shown in Table 13.

**Table 13: Mₙ, M_{w} and PDI regarding dimethylaminopropyl methacrylamide-butyl methacrylate-methyl methacrylate copolymer via GPC analysis**

| **concentration of n-dodecyl mercaptan [%]** | **number-average molecular weight (Mₙ) [kDa]** | **weight-average molecular weight (M_{w}) [kDa]** | **polydispersity index (PDI)** |
|---|---|---|---|
| 1.5 | 41.5 | 173 | 4.17 |
| 0.9 | 64.1 | 254 | 3.97 |
| 0.5 | 71.7 | 281 | 3.92 |
| 0.3 | 81.4 | 305 | 3.75 |

### Dissolution studies

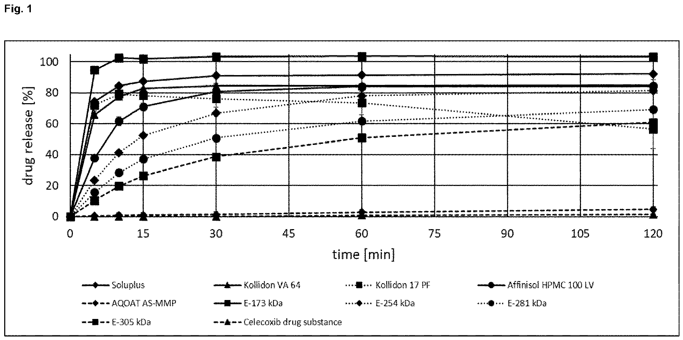

The highest level of drug release for Solid-SNEDDS incorporating celecoxib was achieved by E-173 kDa (Figure 1). Furthermore, this formulation was stable during the entire 120 min of the conducted dissolution test and did not show precipitation at all. Generally, in comparison to PODS incorporating celecoxib, the Solid-SNEDDS presented higher levels of drug release as well as a faster drug release. For the PODS incorporating celecoxib, E-173 kDa and Soluplus® showed the highest drug release of around 88% after 120 min (Figure 2).

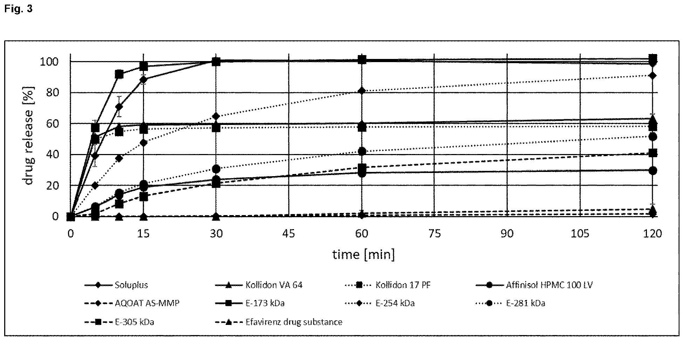

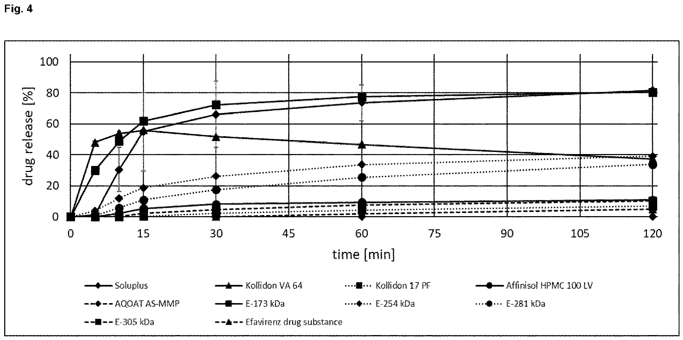

The highest level of drug release for Solid-SNEDDS incorporating efavirenz was achieved by E-173 kDa, closely followed by Soluplus® and E-254 kDa (Figure 3). These three formulations were stable during the entire 120 min of the conducted dissolution test and did not show precipitation at all. Generally, in comparison to PODS incorporating efavirenz, the Solid-SNEDDS presented higher levels of drug release as well as a faster drug release. For the PODS incorporating efavirenz, E-173 kDa and Soluplus® showed the highest drug release of around 80% after 120 min (Figure 4).

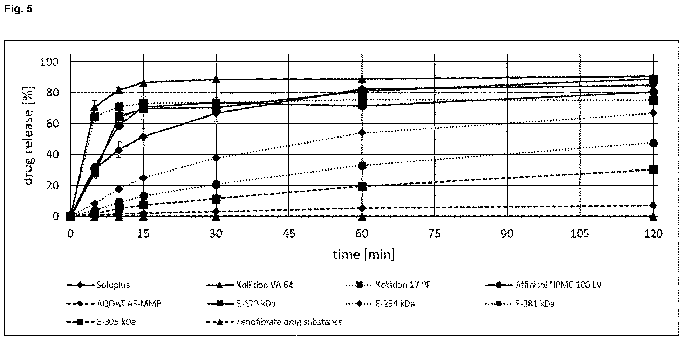

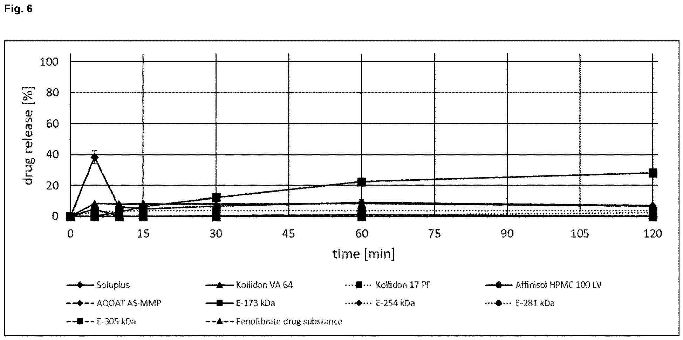

The highest level of drug release for Solid-SNEDDS incorporating fenofibrate was achieved by E-173 kDa and Kollidon® VA 64, closely followed by Soluplus®, Affinisol® HPMC 100 LV and Kollidon® 17 PF (Figure 5). These five formulations were stable during the entire 120 min of the conducted dissolution test and did not show precipitation at all. Generally, in comparison to PODS incorporating fenofibrate, the Solid-SNEDDS presented higher levels of drug release as well as a faster drug release. For the PODS incorporating fenofibrate, E-173 kDa significantly showed the highest drug release of around 30% after 120 min (Figure 6). An initial burst release of fenofibrate was identified for Soluplus® followed by precipitation after 10 min of testing (Figure 6).

### Stability studies

### Appearance

After 3 months of storage under defined and constant conditions (30 °C/65% RH), almost all samples did not show any notable formation of agglomerates and could be refluffed easily. For Solid-SNEDDS manufactured with the polymer Soluplus®, larger agglomerates could be found in samples containing the drug substance celecoxib.

### Dissolution studies (after 3 months of storage)

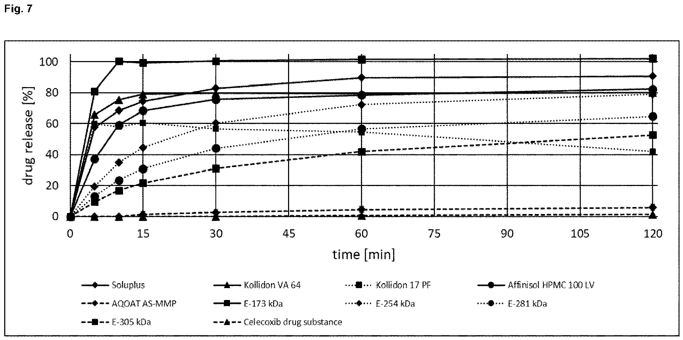

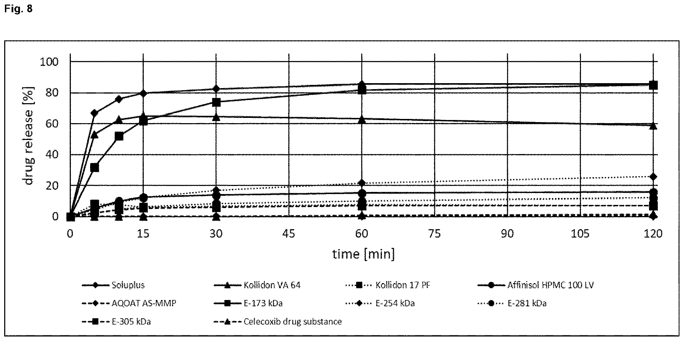

Dissolution tests of Solid-SNEDDS & PODS were performed using the mentioned method for the drug substance celecoxib, and the percentages of drug releases after 120 min were determined (Figures 7 and 8). In comparison to the samples at the time of manufacture depending on the polymer, varying decreases of drug release could be detected (Table 14). AQOAT® AS-MMP did not show any decrease of drug release after 3 months of storage. Apart of this polymer with no decrease in drug release, E-173 kDa presented the lowest relative loss in drug release for Solid-SNEDDS as well as PODS after storage. Except Kollidon® 17 PF, E-281 kDa and E-305 kDa, all Solid-SNEDDS revealed a lower loss of drug release after storage in comparison to the corresponding PODS formulation.

**Table 14: Comparison of drug release regarding Solid-SNEDDS (composition of table 1) & PODS at the time of manufacture and after 3 months of storage incorporating the drug substance celecoxib**

| **polymer** | **drug release Solid-SNEDDS [%]** | **drug release Solid-SNEDDS (after 3 months) [%]** | **absolute loss of drug release (Solid-SNEDDS) [%]** | **relative loss of drug release (Solid-SNEDDS) [%]** | **drug release PODS [%]** | **drug release PODS (after 3 months) [%]** | **absolute loss of drug release (PODS) [%]** | **relative loss of drug release (PODS) [%]** |
|---|---|---|---|---|---|---|---|---|
| Soluplus® | 92.1 | 90.7 | 1.4 | 1.5 | 88.3 | 85.6 | 2.7 | 3.1 |
| Kollidon® VA 64 | 84.8 | 80.0 | 4.8 | 5.7 | 65.7 | 58.8 | 6.9 | 10.5 |
| Kollidon® 17 PF | 56.5 | 42.0 | 14.5 | 25.7 | 9.3 | 7.1 | 2.2 | 23.7 |
| AQOAT® AS-MMP | 4.8 | 6.0 | 0 | 0 | 0.4 | 0.4 | 0 | 0 |
| E-173 kDa | 103.3 | 102.1 | 1.2 | 1.2 | 87.7 | 85.1 | 2.6 | 3.0 |
| E-254 kDa | 81.5 | 79.1 | 2.4 | 2.9 | 27.0 | 25.8 | 1.2 | 4.4 |
| E-281 kDa | 69.1 | 64.8 | 4.3 | 6.2 | 12.4 | 12.1 | 0.3 | 2.4 |
| E-305 kDa | 60.8 | 50.9 | 9.9 | 16.3 | 7.6 | 7.2 | 0.4 | 5.3 |
| Affinisol® HPMC 100 LV | 84.3 | 82.5 | 1.8 | 2.1 | 16.9 | 15.8 | 1.1 | 6.5 |

### Correlation between drug release and glass transition temperature (T_{g})/molecular weight (M_{w})

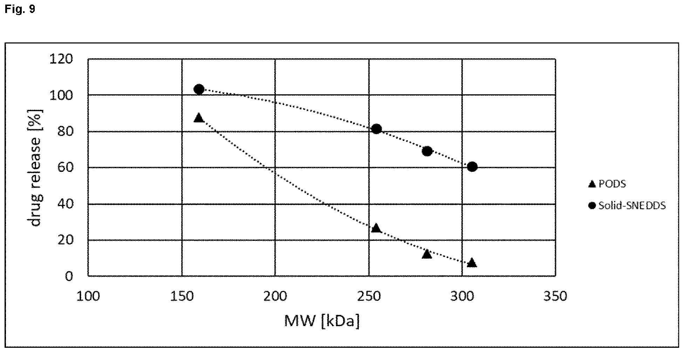

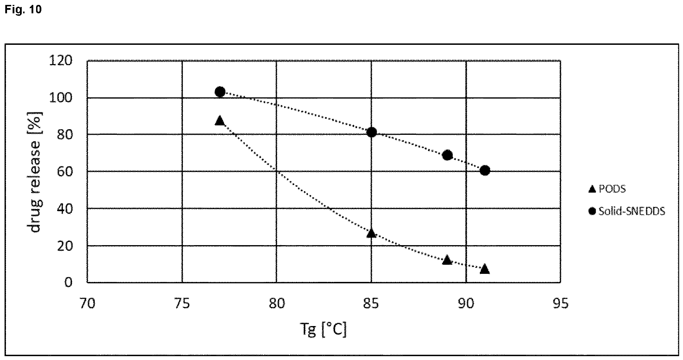

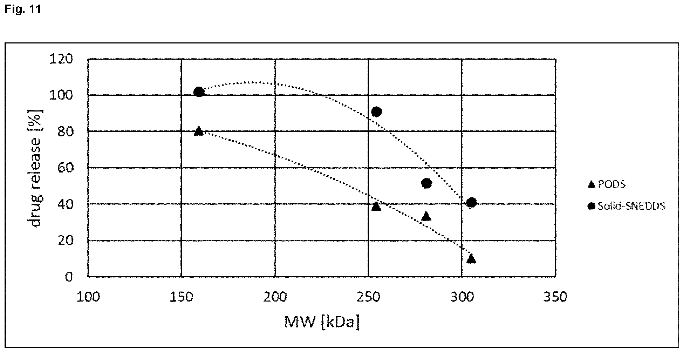

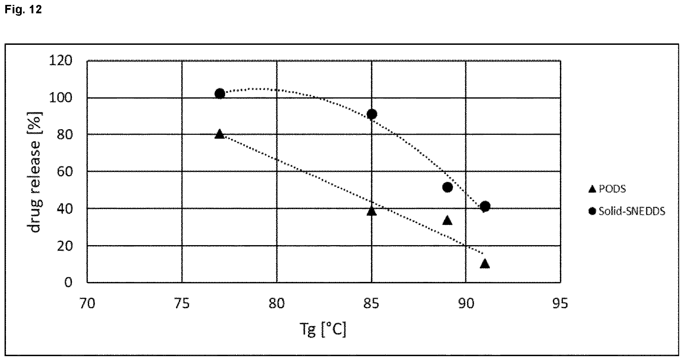

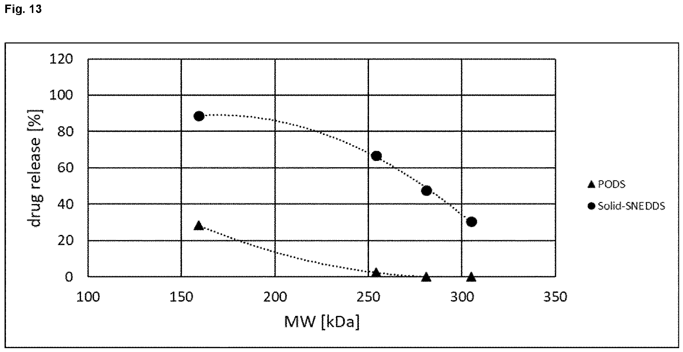

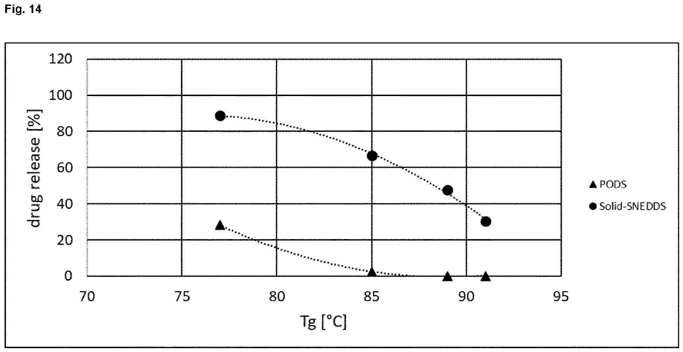

For the copolymers of research studies from Evonik Nutrition & Care GmbH (Darmstadt, Germany), the "Dimethylaminopropyl methacrylamide-butyl methacrylate-methyl methacrylate copolymer (2:1:1, ratios by weight) (E-173 kDa, E-254 kDa, E-281 kDa, E-305 kDa)", a correlation between the drug release and its *T*_{g} or M_{w} is presented. The data (aforementioned) that emphasizes the correlation is illustrated in figures 9 to 14. The data for the drug substances celecoxib and fenofibrate almost revealed a perfect correlation (r² = 1) between drug release and *T*_{g}/M_{w} (Figures 9, 10, 13, and 14).

## Claims

1. Pharmaceutical composition, comprising
(i) at least one methacrylic copolymer comprising units derived from methacrylamide;
(ii) at least one pharmaceutically active ingredient;
(iii) at least one lipid component;
(iv) at least one surfactant;
(v) optionally at least one solvent;
(vi) optionally at least one (meth)acrylic copolymer, different from (i); and
(vii) optionally at least one additive.

2. Pharmaceutical composition according to claim 1, wherein
the at least one methacrylic copolymer comprising units derived from methacrylamide has
i) a weight average molecular weight M_{w} of from 50,000 to 500,000 g/mol; and/or
ii) a glass transition temperature *T*_{g} of from 65 to 100 °C.

3. Pharmaceutical composition according to claim 1 or 2, wherein
the at least one methacrylic copolymer comprising units derived from methacrylamide is a dimethylaminopropyl methacrylamide-butyl methacrylate-methyl methacrylate copolymer.

4. Pharmaceutical composition according to any of the preceding claims, wherein
the at least one methacrylic copolymer comprising units derived from methacrylamide is obtained by radical polymerization of
40 to 60 wt.-% of dimethylaminopropyl methacrylamide,
15 to 35 wt.-% of butyl methacrylate; and
15 to 35 wt.-% of methyl methacrylate, wherein the sum of the monomers is 100 wt.-%, in the presence of at least one initiator and at least one chain-transfer agent.

5. Pharmaceutical composition according to any of the preceding claims, wherein the at least one pharmaceutically active ingredient has a solubility of less than 0.1 mg in 1 ml water at 37 °C and/or is selected from celecoxib, efavirenz and fenofibrate or mixtures thereof.

6. Pharmaceutical composition according to any of the preceding claims, wherein the at least one lipid component is selected from C₆-C₁₂ fatty acid triglycerides; C13-C21 fatty acid triglycerides; propylene glycol dicaprylate / dicaprate; glyceryl tricaprylate / tricaprate; glyceryl triricinoleate; lauric acid triglycerides; glyceryl dibehenate; linoleic acid and oleic acid triglycerides; linoleic acid, oleic acid, and palmitic acid triglycerides; ethyl oleate; isopropyl myristate; monolinoleate triglycerides / diglycerides / monoglycerides; glyceryl tricaprylate / tricaprate / trilaurate; oleic acid; oleic acid and palmitic acid triglycerides; palmitic acid, oleic acid, and linoleic acid triglycerides; oleic acid, linoleic acid, and palmitic acid triglycerides; linoleic acid, oleic acid, and palmitic acid triglycerides; linoleic acid, oleic acid, alpha-linolenic acid, and palmitic acid triglycerides; linoleic acid, oleic acid, and stearic acid triglyceride; glyceryl triacetate; glyceryl tricaprylate; hard fat or any mixtures thereof.

7. Pharmaceutical composition according to any of the preceding claims, wherein the at least one surfactant is selected from polyoxyethylene (23) lauryl ether; polyoxyethylene (2) oleyl ether; glyceryl monooleate; caprylate and caprate monoglycerides/diglycerides; glyceryl monocaprylate; propylene glycol monocaprylate; polyoxyl-35 hydrogenated castor oil; polyoxyl-40 hydrogenated castor oil; lauroyl polyoxyl-32 glycerides; stearoyl polyoxyl-32 glycerides; polyoxyl-15 hydroxystearate; triblock copolymer of polyoxyethylene and polyoxypropylene; oleoyl polyoxyl-6 glycerides; linoleoyl polyoxyl-6 glycerides; lauroyl polyoxyl-6 glycerides; caprylocaproyl polyoxyl-8 glycerides; propylene glycol monolaurate; polyoxyl-40 stearate; diacetylated monoglyceride; polyglyceryl-3 dioleate; sorbitan monolaurate; sorbitan monooleate; sorbitan sesquioleate; sorbitan trioleate; glyceryl monostearate; d-α-tocopherol polyethylene glycol 1000 succinate; polyoxyethylene sorbitan monolaurate; polyoxyethylene sorbitan monostearate; and polyoxyethylene sorbitan monooleate or any mixtures thereof.

8. Pharmaceutical composition according to any of the preceding claims, wherein the at least one solvent is selected from diethylene glycol monoethyl ether, polyethylene glycol 200, polyethylene glycol 400, polyethylene glycol 6000, propane-1,2,3-triol, (z)-octadec-9-enylamine, polypropylene glycol, propylene glycol, 2-pyrrolidone, tetraethylene glycol and diethylene glycol monoethyl ether or any mixtures thereof.

9. Pharmaceutical composition according to any of the preceding claims, wherein the at least one (meth)acrylic copolymer, different from (i), is a methacrylate copolymer obtained by polymerizing 40 to 60 wt.-% methacrylic acid and 60 to 40 wt.-% of ethyl acrylate, based on the total weight of the monomers.

10. Pharmaceutical composition according to any of the preceding claims, wherein the at least one additive is selected from antiadherents; binders; flavors; pigments; disintegrants; glidants; flow regulators; antioxidants; sweeteners; and antistatics; or mixtures thereof.

11. Pharmaceutical composition according to any of the preceding claims, wherein
(i) is present in 40 to 99 wt.-%, based on the total weight of the composition; and
(ii) to (vii) are present in 1 to 60 wt.-%, based on the total weight of the composition, wherein
(ii) is present in 0.1 to 50 wt.-%;
(iii) is present in 5 to 60 wt.-%;
(iv) is present in 1 to 60 wt.-%;
(v) is present in 0 to 35 wt.-%;
(vi) is present in 0 to 84 wt.-%;
(vii) is present in 0 to 10 wt.-%; based on the total weight of components (ii) to (vii), respectively.

12. Pharmaceutical composition according to any of the preceding claims, wherein the pharmaceutical composition is a solid self-nanoemulsifying drug delivery system.

13. The pharmaceutical composition according to any of the preceding claims for use as a medicament.

14. Method of preparing a solid self-nanoemulsifying drug delivery system comprising or consisting of the steps:
providing a self-nanoemulsifying drug delivery system by mixing compounds (ii) to (iv) and optionally (v) of any of claims 1 to 11, and then
applying the obtained self-nanoemulsifying drug delivery system on compound (i) and optionally compounds (vi) and (vii) of any of claims 1 to 11 by hot melt extrusion, spray drying, adsorption, electrospinning, electro spraying, prilling by vibration, granulation or supercritical fluidization to obtain the solid self-nanoemulsifying drug delivery system.

15. Solid self-nanoemulsifying drug delivery system obtained by the method of claim 14.
